# EUROPEAN PATENT SPECIFICATION

(11) **EP 3 678 609 B1**
(45) Date of publication and mention of the grant of the patent: **02.11.2022**
(21) Application number: 18769455.9
(22) Date of filing: 07.09.2018
(51) Int. Cl.: A61F 2/80

(54) **PROSTHETIC LIMB SOCKET**
SCHAFT FÜR GLIEDMASSENPROTHESE
EMBOÎTURE DE MEMBRE ARTIFICIEL

(30) Priority: 08.09.2017 GB 201714498
(43) Date of publication of application: 15.07.2020
(60) Divisional application: 22187669.1
(73) Proprietor: Blatchford Products Limited, Basingstoke Hampshire RG22 4AH (GB)
(72) Inventor: LASZCZAK, Piotr, Southampton Hampshire SO16 2JA (GB); MOSER, David, Southampton Hampshire SO16 7PN (GB); GALLEGO MURILLO, Ana Isabel, London SW2 2TA (GB); MCCARTHY, Joseph Ronald, Fareham Hampshire PO17 6LA (GB); ZAHEDI, Mir Saeed, Barnes London SW13 9BH (GB)
(74) Representative: Jaeger, Michael David
(86) International application number: PCT/GB2018/052550
(87) International publication number: WO 2019/048877

(56) References cited:
- WO-A1-2015/103708
- US-A1- 2010 274 364
- US-A1- 2011 071 647
- US-A1- 2013 123 940
- US-B1- 7 300 466

## Description

### Technical Field

The present invention relates to a socket for a prosthetic limb. In particular, the present invention relates to an adjustable socket for use with a lower limb prosthesis.

### Background to the Invention and Prior Art

A prosthesis can be attached to a residual limb in the following manner. A hollow rigid socket is custom made from a moldable material to accommodate the shape of the residual limb. At the distal end of the socket there is an attachment for attaching the prosthetic device to the socket, such as a pyramid alignment interface. Many amputees use a soft flexible liner as an interface between the residual limb and the socket. Before inserting the residual limb into the socket the liner is donned on the residual limb. The liner provides a resilient cushioning layer to improve comfort for the user. Liners are typically made from an air impermeable material such as silicone or a thermoplastic elastomer gel. Some liners, whilst being made from air impermeable material, are perforated to allow for flow of fluid from the inner surface of the liner to the outer surface thereof.

An intimate fit is required between the residual limb, the liner and the socket. This close fit is required to prevent relative motion between the socket, liner and residual limb, to prevent irritation of the skin and other soft tissue of the residual limb. The intimate fit also better distributes forces on the residual limb when weight is applied to the prosthesis via the socket. For a lower limb prosthesis, this will occur when the prosthesis is in contact with the ground, for example when standing and during the stance phase of the gait cycle.

During the course of the day, there will be changes in the volume of an amputee's residual limb and its shape (due to movement of fluid and soft tissue). This variation depends on many factors, including but not limited to the individual residual limb's characteristics, medical conditioning, the environment and types of activity undertaken by the amputee, etc.. For example, during walking the socket will exert varying pressure on the residual limb. When there is no activity, for example when the prosthetic limb is not worn and during the night, the original shape of the limb will be restored. It is also known that residual limb volume will change considerably when worn during the course of the day and with periods of activity and rest. In order to accommodate this volume variation the amputee may wear a plurality of socks over the liner (or next to skin, when a liner is not worn), which can be removed or supplemented during the course of the day in order to maintain a comfortable and effective fit between the residual limb and the socket. At present this adjustment of fit is based largely on the skill and judgement of the amputee. However, due to underlying health issues some amputees may experience sensory loss within the residual limb, which makes these adjustment decisions more difficult. It is therefore an aim of the present invention to overcome some of these difficulties.

Another way of accommodating the variation in the volume of the residual limb is by altering the size and shape of the socket or the internal cavity thereof.

US-A1-2006/009860 describes an adjustable prosthetic socket comprising a cup, a lateral wall, and a brim. The cup includes a lateral cutaway portion, a lower closed portion, and an upper open portion. The brim is adapted for adjustable mounting in said cup via said upper open portion while the lateral wall is adapted for adjustable mounting in said lateral cutaway.

US-A1-2013/218296 describes a socket and suspension system. Moving fluid in or out of an area between a liner and the socket causes the liner to push away from the socket wall or pull into the socket wall thereby changing the volume inside the socket.

US-A1-2015/119997 describes an adjustable prosthetic limb socket system having a partial rigid support, a non-elastic, flexible support and adjustment means.

WO-A1-2015/013560 describes an adjustable socket for a prosthetic limb including an adjustment mechanism to adjust a volume of the cavity to accommodate an increase or decrease in a volume of the user's residual limb.

WO-A1-2015/013506 describes an adjustable socket for a prosthesis including adjustable cells each capable of bearing a portion of a weight of the limb.

US-A1-2011/071647 is considered to represent the closest prior art for the present invention, it describes adjustable prosthetic interfaces and related systems and methods. Prosthesis devices can include sockets having adjustable features. In one example, a socket includes one or more panels that can move outwardly or inwardly relative to a receptacle portion of the socket. The panels can be moved by tightening a tensioning line.

US-A1-2010/274364 describes a prosthesis system including a reconfigurable socket. The socket changes configurations to adjust the socket fit. The socket includes a socket main body with a window and a panel positioned in the window. The panel and the socket main body cooperate to define a cavity for receiving a residual limb. A lacing system is coupled to both the socket main body and the panel and moves the panel with respect to the socket main body to adjust a volume of the cavity. A tensioning mechanism holds the lacing system to position the adjustment panel. The prosthesis system also allows for the escape of moisture from within the cavity.

The present invention provides an improved adjustable prosthetic socket.

### Summary of Invention

According to a first aspect of the invention, there is provided a prosthetic limb socket comprising:
a rigid support member comprising a first cantilevered support portion extending around a majority of a circumference of the socket and a second cantilevered support portion opposite the first cantilevered support portion and defining a pair of windows between lateral edges of the first and second cantilevered support portions;
first and second adjustable panels, each panel being disposed in one of the pair of windows between the first and second cantilevered support portions; and
means for adjusting a radial position of the panels relative to the support portions,
wherein the panels are held in position between the support portions by the means for adjusting.

The socket may be a transfemoral or knee disarticulation socket for a transfemoral or knee disarticulation residual limb.

The first support portion may extend around a medial portion of the circumference of the socket; the first panel may extend along an anterior lateral portion of the socket; the second panel may extend along a posterior lateral portion of the socket; and the second support portion may extend along a lateral portion of the socket.

The first and second panels may extend proximally beyond the second support portion.

The socket may be a transfemoral or knee disarticulation socket for a transfemoral or knee disarticulation residual limb respectively and the plurality of support portions may comprise first, second and third cantilevered support portions, the support portions defining between their lateral edges first, second and third windows. The plurality of adjustable panels may comprise first, second and third adjustable panels, each panel being disposed in one of the first, second and third windows. Alternatively, the socket may comprise fourth or fifth support portions, windows and adjustable panels.

The panels may comprise a rigid or semi-rigid outer shell and a resiliently compressible inner portion.

The inner portion may comprise a strip of ethylene-vinyl acetate.

In combination the support portions and panels may extend around the circumference of the socket.

The means for adjusting a radial position of the panels relative to the support portions may comprise a cord or cords traversing the support portions and the panels.

The cord is or the cords may be disposed on an outer surface of the support portions or embedded within an outer shell of the support portions.

The cords may comprise a first cord and a second cord.

The socket may further comprise means for drawing the cord or cords radially inwards.

The means for drawing the cord or cords radially inwards may comprise a ratchet mechanism.

The ratchet mechanism may comprise first and second ratchet reels, each ratchet reel drawing in one of the first and a second cords, the first and second cords being spaced distally and proximally of each other along the support portions.

The means for drawing the cord radially inwards may comprise a motor.

According to a second aspect of the invention there is provided a prosthetic limb socket comprising:
a rigid-walled support member as described above;
a plurality of sensors within the support member; and
means for adapting one or more of:
   an interior volume of the support member;
   a temperature within the support member;
   oxygen levels within the support member; and
   a pressure and/or shear within the support member, and
communications modules for communicating between the sensors and the means for adapting.

The means for adapting an interior volume of the support member may comprise one or more of: a rigid support member and a plurality of support portions as described above; a ski-boot type adjustable buckle and buckle hook; and one or more adjustable volume bladders.

The means for adapting a temperature within the support member comprise one or more of: a thermoelectric element; a fan; and an extraction port in a wall of the socket.

The sensors may sense one or more of: temperature; humidity; oxygen levels; pressure; shear/friction; bacteria; movement; proximity; and displacement.

### Brief Description of the Drawings

The present invention will now be described by way of example only, and with reference to the accompanying drawings in which:
Figure 1A is an anterior view of a transfemoral socket according to a first embodiment of the invention;
Figure 1B is a medial view of the socket of Figure 1A;
Figure 1C is an anterior view of the socket of Figure 1A;
Figure 1D is a lateral view of the socket of Figure 1A;
Figure 1E is a plan view of the socket of Figure 1A;
Figure 1F is a plan view of the socket of Figure 1A with the internal liner and foam pads removed;
Figure 2 is a front view of a transfemoral or knee disarticulation socket;
Figure 3 is a front view of a transfemoral or knee disarticulation socket;
Figure 4 is a front view of a transfemoral or knee disarticulation socket;
Figure 5 is a front view of a transtibial or Chopart socket;
Figure 6 is a front view of a further transtibial or Chopart socket according to a further embodiment of the invention;
Figure 7 is a front view of further sockets according to further embodiment of the invention;
Figure 8 is a schematic representation of a powered adjustable socket in accordance with a further embodiment of the invention;
Figure 9 is a schematic representation of a system including the powered adjustable socket of Figure 8; and
Figure 10 is a schematic representation of a powered adjustable socket in accordance with a further embodiment of the invention.

### Detailed Description of the Drawings

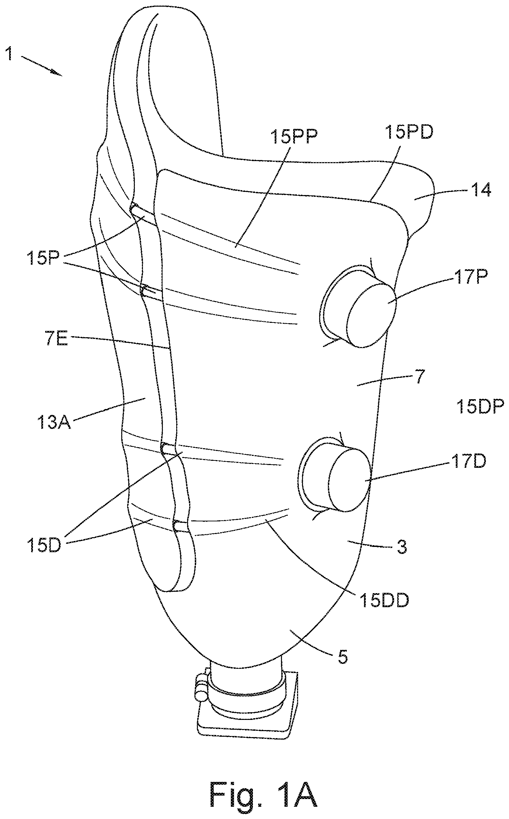

Figures 1A-D show side views of a socket 1 according to a first embodiment of the invention for use with a residual transfemoral limb. A similar socket can be used for a knee disarticulation. The socket 1 is for an amputee's right leg (residuum) and a mirror image socket can be used for an amputee's left residuum. The socket 1 comprises a rigid support member 3 having a distal base portion 5 and first 7 and second 9 cantilevered support portions or struts extending proximally from the base portion 5. As can be seen from each of Figures 1A-D, the first support portion 7 extends around a majority of a circumference of the socket 1, to surround a majority of a residual limb inserted into the socket 1.

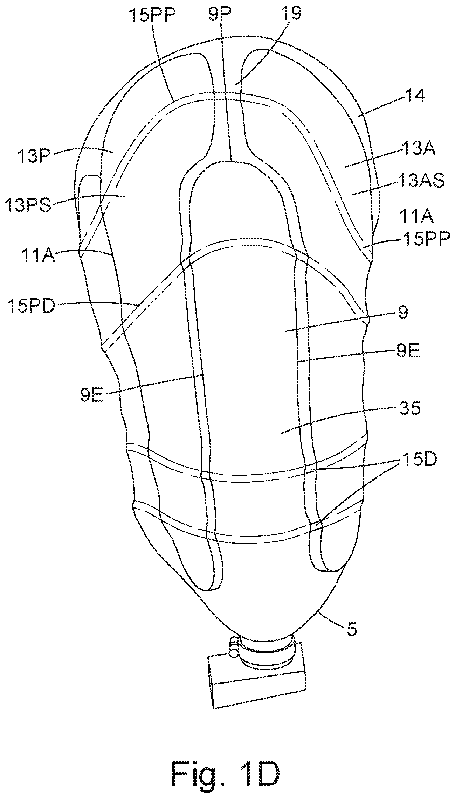

In particular, the first support portion 7 begins at the antero-lateral side of the socket 1, continues medially and posteriorly and ends at the lateral posterior side of the socket 1. The support portion 7 subtends an angle of around 240° to a central axis of the socket 1. In contrast to the support portion 7, the second support portion 9 which is opposite the first support portion 7, is only visible in Figure 1D and only extends circumferentially over a portion of the lateral side of the socket 1. The second support portion 9 subtends an angle of around 50° to a central axis of the socket 1. (In this context these terms have the following meaning: anterior is the front of the socket 1 when worn by the amputee; medial is the inside of the socket 1 when worn, i.e., the side facing the amputee's other limb; posterior is the rear of the socket 7 when worn; and lateral is the outside of the socket 1 when worn by an amputee.)

Since the first 7 and second 9 support portions extend proximally from the distal base portion 5, they define between their lateral edges 7E, 9E windows 11A, 11P. One window 11A is a lateral anterior window and the other window 11P is a lateral posterior window. Disposed within the windows 11A, 11P are respective lateral anterior 13A and lateral posterior 13P adjustable panels. These panels 13A, 13P may be made of a rigid or a semi-rigid material. The lateral anterior 13A and lateral posterior 13P panels are generally shaped to correspond to the shape of the respective windows 11A, 11P, to substantially fill the windows, but are separate pieces to the rigid support member 3. As can be seen from Figure 1D the lateral anterior 13A and lateral posterior 13P rigid panels are hockey stick shaped and extend above a proximal end 9P of the second support portion 9 and are arced towards each other above the proximal end 9P of the second support portion 9.

The outer shell 3S of the rigid support member 3 and the outer shells 13AS, 13PS of the lateral anterior 13A and posterior 13P adjustable panels are made of carbon fibre, glass reinforced plastic or another rigid material that can be molded to the shape of a residual limb. Alternatively, the rigid support member 3 and the outer shells 13AS, 13PS of the lateral anterior 13A and posterior 13P rigid panels may be manufactured from scans or casts of the residual limb using additive manufacturing technique, including both single and multimaterial techniques. The outer shells 3S, 13AS, 13PS are shaped to conform to the outer shape of the residual limb of the amputee following the standard technique used to form sockets, namely casting a negative cast around the residual limb, casting a second positive cast having the shape of the residual limb and using the positive cast to form the outer shells 3S, 13AS, 13PS. The outer shells 3S, 13AS, 13PS may be made by forming a single shell corresponding to the shape of the residual limb and then cutting out the anterior 13A and posterior 13P adjustable panels to leave behind the rigid support member 3, or by separately forming these three parts 3, 13A, 13B.

The panels and or other portions of the socket may also be made of adjustable structural materials for example based on material "jamming" principles. In such embodiment, the panel(s) consist of granular material contained in a bag, when the air contained within the bag is evacuated the granular material are brought together, "jamming" to create a rigid shape conforming to the interfacing structures (e.g. the residual limb and/or socket). Similarly, a few layers of socks each with fabrics woven in different direction and contained in an airtight bag would "jam" when air was evacuated from the said bag.

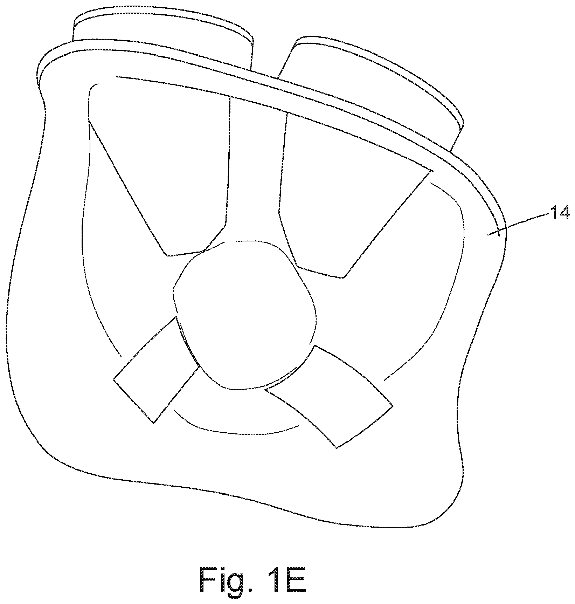

Figure 1E is a plan view of the socket of Figure 1A. Mounted on the inside of the support portions 7, 9 and lateral anterior 13A and posterior 13P rigid panels are strips of expanded cross-linked ethylene-vinyl acetate (EVA). EVA is an elastomeric polymer that produces materials which are "rubber-like" in softness and flexibility. The EVA strips act to cushion the limb within the support portions 7, 9 and panels 13A, 13P, while simultaneously providing compression and hence control over the prosthesis. Alternatively, strips of Pe-Lite foam, viscoelastic material such as silicone strips, or other foam with cushioning properties can be used to cushion the limb.

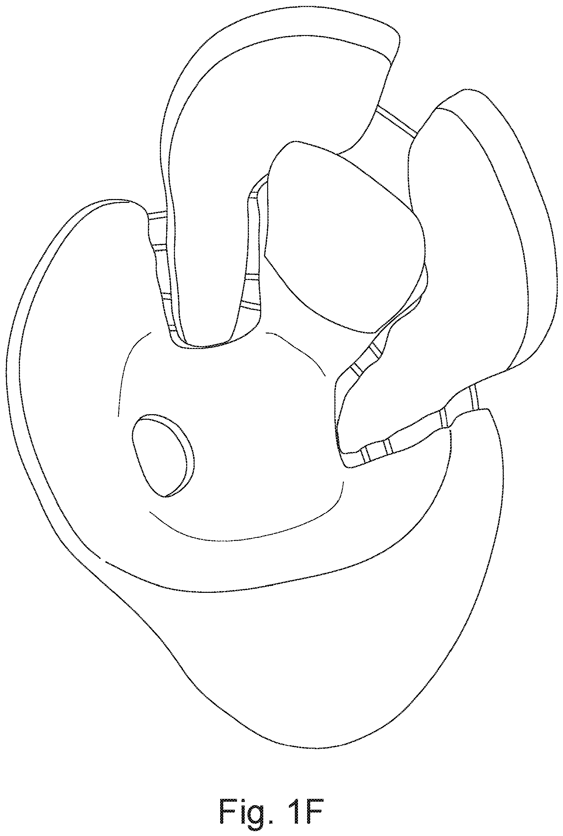

An internal flexible liner is disposed within the socket. The liner is made from a material such as Northvane (RTM) which is manufactured by North Sea Plastics (RTM) Ltd. Figure 1F is a plan view of the socket of Figure 1A similar to the view shown in Figure 1E but with the inner liner 14 removed. This internal flexible liner is typically used when the socket is part of a vacuum suspension system. A vacuum suspension system is commonly used by amputees whose limbs are subject to large volume fluctuation, since the negative pressure (vacuum) reduces volume fluctuation. Hence in some embodiments of the invention no internal flexible liner is required.

The lateral anterior 13A and lateral posterior 13P adjustable panels are held in position on the rigid support member 3 by means of a pair of cords or laces 15D, 15P traversing the lateral windows 11A, 11P. These cords serve as means for adjusting a radial position of the panels 13A, 13P relative to the support portions 7, 9. The cords 15D, 15P have the structure of a Bowden cable, having an inner cable which can move easily relative to a hollow outer cable housing which is fixed to the wall of the support portions 7, 9 and lateral rigid panels 13A, 13P. In particular, the cords comprise a first, proximal cord 15P and a second, distal cord 15D. The cords may also be actuated using, for example, electroactive polymers or shape memory materials. Aside from radial adjustment, the arrangement of cords may also be used to rotate and/or adjust the longitudinal or medial lateral position of the panels to better fit with the residual anatomy. This positional adjustment of the support panels is particularly important, for example, with a child socket and with a growing residual limb.

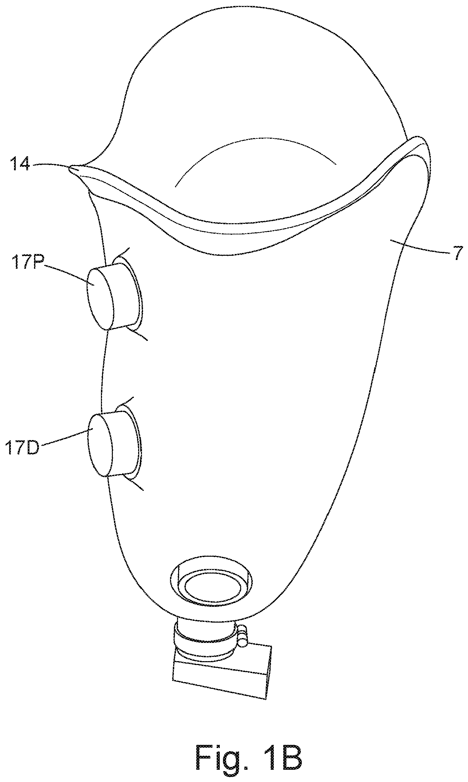

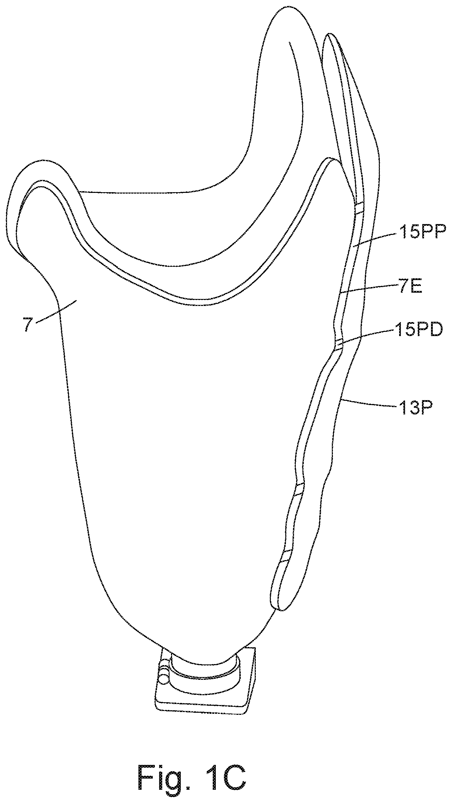

The proximal cord 15P passes around a partial circumference of a proximal portion of the socket 1 in the following manner. A proximal reel 17P (see Figures 1A and 1B) is mounted on the medial anterior portion of the first support portion 7 of the rigid support member 3. The proximal cord 15P passes around the proximal reel 17P and then continues as a proximal portion 15PP and a distal portion 15PD, extending from the proximal reel 17P and running substantially in parallel around the socket 1. As can be seen from Figure 1A, both the proximal 15PP and the distal 15PD portions of the proximal cord 15P run anteriorly to the front of the socket 1 and then continue round the socket 1 around the lateral side of the socket 1 until they pass from the first support portion 7 to the lateral anterior rigid panel 13A. As can be seen in Figure 1D, the proximal portion 15PP of the proximal cord 15P rises proximally up the anterior panel 13A to run from the anterior panel 13A to the posterior panel 13P above the proximal end 9P of the second support portion 9 and across a gap 19 between facing upper ends of the anterior 13A and posterior 13P panels. Once on the posterior panel 13P, the proximal portion 15PP of the proximal cord 15P sinks distally down the posterior panel 13P to run from the posterior panel 13P to the posterior portion of the first support portion 7, as shown in Figure 1C. Once on the first support portion 7 the proximal cord 15P descends downwards and curves backwards to begin the distal portion 15PD of the proximal cord 15P. On its return route to the proximal reel 17P, the distal portion 15PD of the proximal cord 15P rises gently proximally up the first support portion 7 until it crosses to the posterior panel 13P (see Figure 1C) and from there to second support portion 9 of the rigid support member 3. From the second support portion 9 the distal portion 15PD of the proximal cord 15P falls gently distally and passes from the second support portion 9 to the anterior panel 13A and continues to run anteriorly and distally until it once again reaches the proximal reel 17P (see Figure 1A).

The distal cord 15D passes around a partial circumference of a distal portion of the socket 1 in a similar manner. A distal reel 17D (see Figures 1A and 1B) is mounted on the medial anterior portion of the first support portion 7 of the rigid support member 3 distally of the proximal reel 17P. The distal cord 15D passes around the distal reel 17D and then continues as a proximal portion 15DP and a distal portion 15DD, extending from the distal reel 17D and running substantially in parallel around the socket 1. As can be seen from Figure 1A, both the proximal 15DP and the distal 15DD portions of the distal cord 15D run anteriorly to the front of the socket 1 and then continue round the socket 1 around the lateral side of the socket 1 (see Figure 1D) until they pass from the first support portion 7 to the lateral anterior adjustable panel 13A. As can be seen in Figures 1A and 1D, the proximal portion 15DP of the distal cord 15D passes from the lateral anterior panel 13A to the second support portion 9, on to the lateral posterior panel 13P and then on to the first support portion 7. As can been seen in Figure 1C, once on the first support portion 7 the proximal portion 15DP of the distal cord 15D descends downwards and curves backwards to begin the distal portion 15DD of the distal cord 15D. On its return route to the distal reel 17D, the distal portion 15DD of the distal cord 15D passes back from the first support portion 7 to the lateral posterior panel 13P and from there to second support portion 9 of the rigid support member 3. From the second support portion 9 the distal portion 15DD of the distal cord 15D passes to the lateral anterior panel 13A and continues to run anteriorly until it once again reaches the distal reel 17D.

The proximal 17P and distal 17D reels serve as a means for drawing the cords 15P, 15D radially inwards. Each reel 17P, 17D includes a releasable ratchet mechanism for tightening and holding the cords 15P, 15D in place. Such ratchet mechanisms are produced by Boa Technology, Inc. and marketed under their Boa (RTM) closure system brand. In this manner the cords 15P, 15D can be tightened, locked in place and released during the course of the day as the amputee adjusts the interior volume of the socket 1. The amputee may make the adjustment, for example, for specific activities requiring more control or comfort. This may be a guided adjustment based on pressure/shear, circumferential measurements using for example a RAG (red, amber green) system, displayed in the liner using wearable electronics or wirelessly using a smart phone app. Alternatively the adjustment may be fully automated by actuating the cords which in turn alters the position of the adjustable panels 13A, 13B.

In use, when the proximal reel 17P is turned to tighten the proximal cord 15P, the proximal portion of socket 1 is drawn inwards around the trochanter of the femur. Similarly, when the distal reel 17D is turned to tighten the distal cord 15D, the distal portion of socket 1 is drawn inwards around the soft tissue at the end of the residuum.

In a further embodiment, instead of the cords 15P, 15D passing around and being tightened by manually tightenable reels 17P, 17D, a motor may be used to automatically or semi-automatically tighten the cords 15P, 15D. This tightening may be performed in response to signals received from sensors incorporated into the sockets, as is explained below.

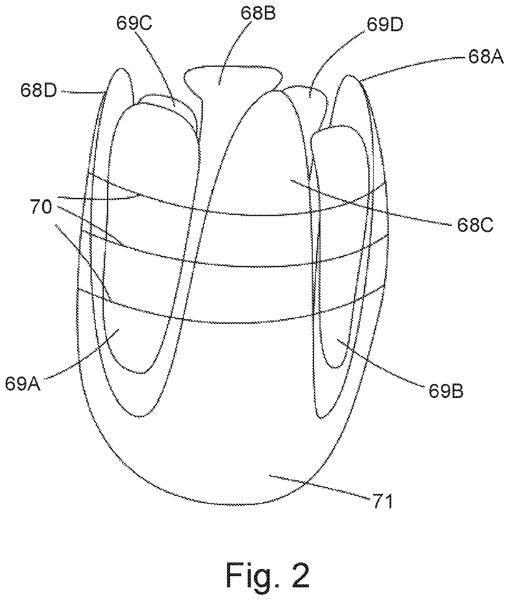

Figure 2 shows a front view of a transfemoral or knee disarticulation socket 71. The socket is for an amputee's right leg (residuum). It contains fixed support portions 68A, 68B, 68C and 68D, located on the medial, posterior, anterior and lateral aspects of the prosthesis, respectively. The support portions are attached to the distal end of the socket 71 and extend proximally. Each support portion subtends an angle of around 50° to a central axis of the socket 71. The posterior support portion 68B curves slightly outwards at its proximal end, and extends circumferentially, thus providing a seating area to provide the amputee with additional support. The socket 71 also contains adjustable panels 69A, 69B, 69C, 69D that are located on the anterior-lateral, anterior-medial, posterior-lateral, posterior-medial aspects of the socket, respectively. The extent to which the panels 69A, 69B, 69C, 69D protrude into the socket is controlled with chords 70. At least two chords, one proximally, and one distally are guided around the entire circumference of the socket 71. Therefore two ratchet mechanisms, one located proximally, and one distally, can control the protrusion of all adjustable panels at once.

In another arrangement (not shown), posterior support portion 68B and medial support portion 68B of Figure 2 are joined together proximally. The area of the socket brim where support portions 68A and 68B are joined curves slightly outwards and extends circumferentially, thus providing a larger seating area than that provided by the socket 71 of Figure 2 and thereby giving the amputee additional support. In this arrangement, the socket contains a window for a panel similar to panel 69D. It also contains panel similar to panel 69D, however such a panel would extend less longitudinally than panel 69D in the previous arrangement. The other elements of the socket remain the same as in Figure 2.

In another arrangement, posterior support portion 68B and medial support portion 68A are joined together into a single support portion that subtends an angle of around 140° to a central axis of the socket 71. The support portion curves outwards at its proximal end, thus providing a seating area and giving an amputee additional support. In this arrangement, the socket does not contain panel 69D shown in Figure 2. The other elements of the socket remain the same as in Figure 2.

In another arrangement, cords 70 can be used to control the position of some of the panels individually.

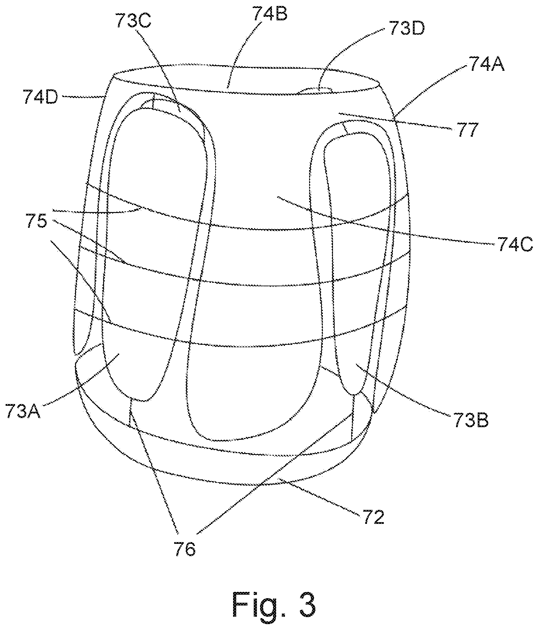

Figure 3 shows a front view of a transfemoral or knee disarticulation socket 72. As with the previous arrangement, the socket 72 is for an amputee's right leg (residuum). It contains fixed support portion 74B, which is located at the posterior aspect of the socket. The support portion is attached to the distal end of the socket 72 and extends proximally. At the proximal end, the support portion joins with the socket brim 77, which extends the entire circumference of the socket. The socket also contains support portions 74A, 74C and 74D, located on the medial, anterior and lateral aspects of the prosthesis, respectively. The support portions 74A, 74C and 74D extend from the brim distally. Each support portions 74 subtends an angle of around 50° to a central axis of the socket 72. The brim 77 curves slightly outside at the posterior-medial location, thus providing a seating area, thereby giving the amputee additional support. The socket also contains adjustable panels 73A, 73B, 73C, 73D that are located on the anterior-lateral, anterior-medial, posterior-lateral, posterior-medial aspects of the socket, respectively. The extent to which the panels protrude into the socket 72 can be controlled with horizontal chords 75 or vertical chords 76. At least two horizontal chords 75, one proximally, and one distally are guided around the entire circumference of the socket 72. Therefore two ratchet mechanisms, one located proximally, and one distally, can control the protrusion of all adjustable panels at once.

In another arrangement, any of the support portion 74A, 74C and 74D extend distally and join the distal portion of the socket 72. Any of the adjustable panels 73A, 73B, 73C and 73D may be removed and substituted with a closed, fixed strut.

In another arrangement, brim 77 may be split, so that it forms a discontinuous hoop. This enables adjusting the brim tightness through means of, for example, ratchets, Velcro (RTM) straps or chords.

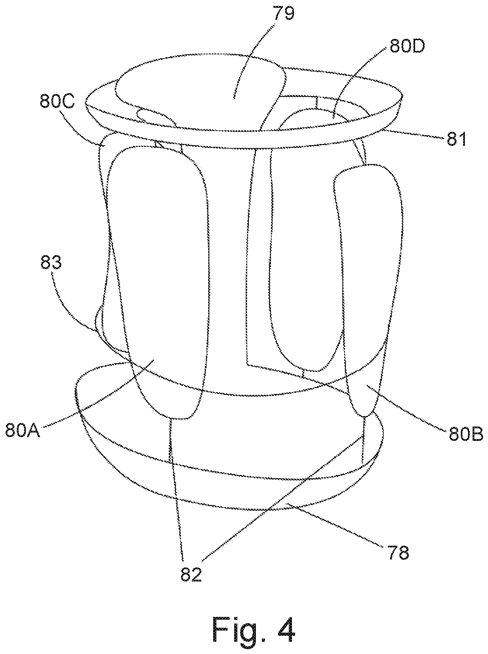

Figure 4 shows a front view of a transfemoral or knee disarticulation socket 78. The socket 78 is for an amputee's right leg (residuum). The socket 78 comprises fixed support portion 79, which is located at the posterior aspect of the socket and which is attached at its distal end of the socket and extends proximally. The support portion 79 subtends an angle of around 60° to a central axis of the socket 78. At the proximal end, the support portion joins with the socket brim 81, made of hard rigid material, which extends the entire circumference of the socket. The brim 81 and strut 79 curve slightly outside at the posterior-medial location, thus providing seating area, giving amputee additional support. The socket also contains adjustable panels 80A, 80B, 80C, 80D that are located on the anterior-lateral, anterior-medial, posterior-lateral, posterior-medial aspects of the socket, respectively. The panels' protrusion into the socket can be controlled with horizontal chords 82. Optionally, horizontal chord 83 can be used to control the protrusion of panels 80A, 80B, 80C, 80D.

In another arrangement, support portion 79 may be located at posterior-medial aspect of the socket. In this arrangement, there is no posterior-medial adjustable panel. The other elements of the socket remain the same as in Figure 4.

In another arrangement, brim 81 may be split at an arbitrary location, so that it forms a discontinuous hoop. This enables adjusting the brim tightness through means of, for example, ratchets, Velcro (RTM) straps or chords.

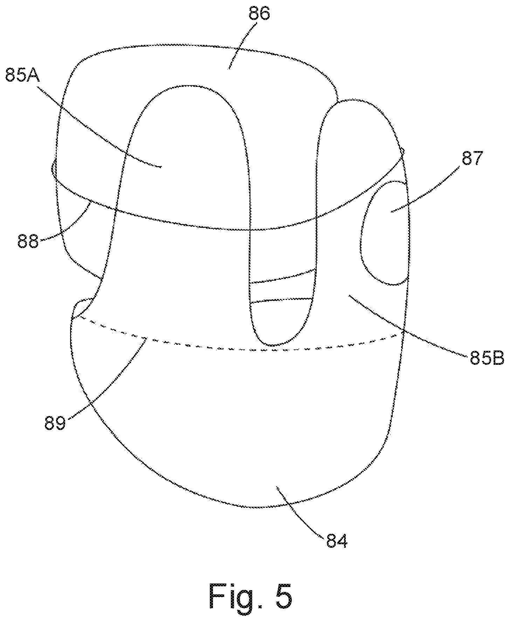

Figure 5 shows a front view of a transtibial or Chopart socket 84. The socket is for an amputee's left leg (residuum). It contains fixed support portions 85A and 85B, which are located at the anterior-medial and anterior-lateral aspects of the socket. The support portions 85A and 85B are attached to the distal portion of the socket and extend proximally, until the patella tendon level. The support portions 85 subtend an angle of around 90° to a central axis of the socket 84. There is a window 87 in the support portion 85b, to provide pressure relief for the fibula head. The window 91 between support portions 85A and 85B provides relief to the tibial crest. The socket 84 also incorporates an adjustable panel 86, which subtends an angle of around 90° to a central axis of the socket 84. The protrusion of the panel into the socket 84 can be controlled with chord 88.

Although support portions 85 are rigid themselves, they are hinged around the dashed line 89. The hinging can be achieved in numerous ways:
- The cross-sectional area of the socket around line 89 is thinner;
- A more flexible material is placed around line 89;
- Different layup of the fibres in composite materials (e.g. carbon-fibre) is employed around line 89;
- Proportions of composite materials is varied around line 89;
- A mechanical hinge is placed around line 89; and/or
- If the socket was manufactured with an additive manufacturing technique, this could be achieved by using different slicing techniques, fillers, multimaterial blends and structures, variation in lattices and relative content in the material varied around the line 89.

The hinging permits controlling protrusion of support portions into the socket. This can be controlled with chord 88. This can be the same or different chord from that controlling adjustable panel 86.

In another arrangement, adjustable panel 86 can be attached to the distal portion of the socket 84 forming a support portion. The support portion is hinged around the same level as the other support portions 85A and 85B (i.e., around line 89). Methods of hinging are the same as listed above.

In another arrangement, support portions 85 extend further proximally, above an amputee's femoral condyles, and curve slightly towards the inside of the socket. This forms supra-condylar suspension, preventing the socket from falling off during swing. These "supracondyles" are hinged at the level of patella tendon, which can be achieved in the same fashion as described above. Therefore, the "supracondyles" can be brought in and out of the socket, providing more or less suspension. The action of bringing the "supracondyles" in and out of the socket (i.e. their actuation) can be achieved with chords, or other actuation methods described below.

In all embodiments, support portions may exhibit variable flexibility. Typically, the support portions would be more rigid distally and less rigid proximally. The variation in the socket flexibility can be achieved with the following methods:
- The cross-sectional area of the socket around line 89 is thinner;
- Variation in thickness;
- Variation in material;
- Variation in layup of the fibres in composite materials (e.g., carbon fibre);
- Variation in material proportions;
- If the socket is manufactured using an additive manufacturing technique, this could be achieved by using different slicing techniques, fillers, multimaterial blends and structures, variation in lattices and relative content in the material.

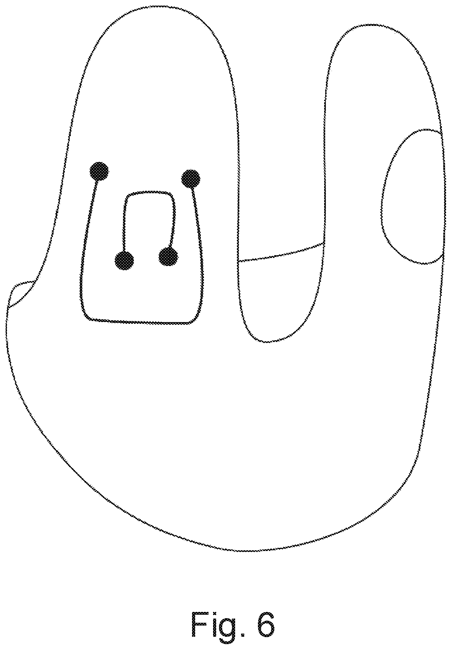

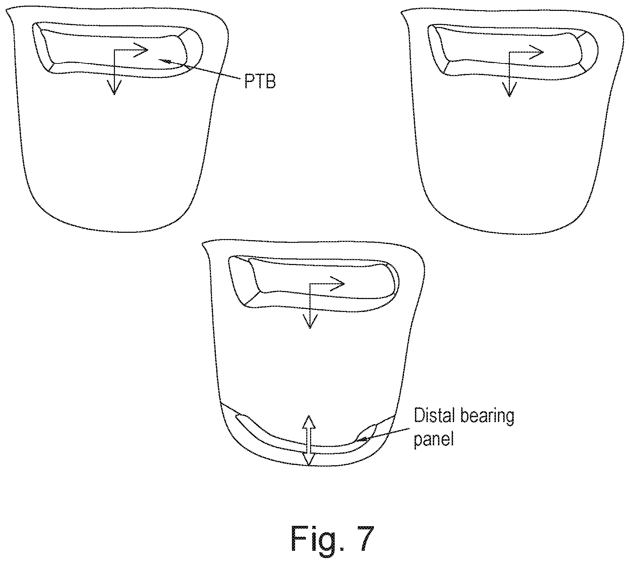

In another embodiment, support portions may contain cut-ins, such as those presented in Figure 6. The cut-ins can be actuated, to allow for control of the socket shape. In another embodiment shown in Figure 7 the location of the adjustable panels can be modified, in the plane of the socket (rather than protruding into or out of the socket 7). This can be achieved for example using straps, ratchets or chords.

Flexion sensors may be placed in the support portions. Flexion measurement may be used, for example, for feedback to the actuators flexing the support portions. An example of such a sensor is optical fibres that are woven into the layup of the support portion. In such sensors a beam of light is injected into the fibre and a detector is placed at the other fibre end. The transduction mechanism relies in the change of light beam properties (e.g., light wavelength), upon flexion of the strut, as measured by the detector.

### Suspension methods

The amputee's residuum could be suspended in the socket in a range of different ways.

With an inner liner 14 - Such a liner allows for generation of vacuum and hence suspending the residuum with either suction or elevated vacuum methods. As an alternative, the liner could be made of a more flexible material and could be moulded over the support portions, thereby forming a single part with the rest of the socket.

With a regular silicone or gel liner and existing suspension techniques such as pin lock, magnetic lock, Velcro (RTM) lock, suction or vacuum suspension. In such case, the liner should feature control of the circumferential stretch, which should vary depending on the tissue viability of the amputee. Amputees with firm tissues would be fitted with more stretchy material (around the circumference). Amputees with more compromised, flaccid tissue, would be fitted with liners limiting the circumferential stretch. This is to ensure that once the pressure is applied through the adjustable panels to the stump, the tissue that is pushed away does not 'bulge out' through the socket 'windows' or through the fixed support portions. Therefore, a liner with limited circumferential stretch acts as a medium of 'containing' the tissue. This stretch may be selectively adjustable in different regions of the liner using actuatable threads, the stretch may also be measurable using stretch sensor, piezo resistive polymers etc.

With a 'hammocking' liner - Such a liner would be attached around the brim of the socket, and the residuum would be supported as if it was in a hammock. This concept is particularly applicable to the socket shown in Figure 4. Different performance can be achieved by controlling the liner's longitudinal stretch across the residuum longitudinal axis. Such a liner can be made of silicone, but could equally be made of fabrics, therefore providing additional air circulation, reducing sweating and improving the overall microclimate of the residuum.

Both liners and sockets may be coated with additives to achieve specific performance. For instance, the liners and sockets may be coated with thermochromic additive, i.e., a material which changes its colour due to a change in temperature. Preferably, the coating should be close to the skin or even at a surface directly contacting the skin. The thermochromic coating may be used as a method of sensing temperature, taking advantage of the change of colour due to a change in temperature. It is known that elevated temperatures can produce discomfort, may cause sweating and thus adversely affect the residuum microclimate. Thermochromic compounds can be tailored to transition from one colour to another at a set temperature (the colour change being reversible). By embedding these compounds in the soft lining of the socket or a liner that can show a colour change, it is possible to indicate to a user an elevated stump temperature so that the user can take appropriate action. In addition such thermochromic additives can be added to a fabric applied to a liner.

Another coating that can be applied to the liner and/or socket is piezochromic additive, i.e., a material which changes its colour when pressure is applied. The lining of a socket or a liner can be formulated with piezochromic pigments such that pressure points are visually highlighted. These additives or compounds can be reversible or irreversible and can be tailored to a specific pressure. These additives can also be added to the fabric.

The concepts of coating with piezochromic or thermochromic compounds may be beneficial in daily routines, but also in diagnostics. For instance, in home settings amputees can determine the intensity of the activities they perform and how these activities affect their residuum status. Therefore feedback in the form of the liner/socket colour can be used in the decision making process as to when to rest the residuum. This is particularly important for neuropathic patients, who have impaired sensation. In a clinical setting, prosthetists frequently perform socket diagnostics and seek locations where sockets are either not in contact with residuum, or where excess loading is applied. This is typically performed using sockets made of a transparent material. Having liners or sockets coated with a heat- or load-sensitive additive can greatly assist with identification of those locations.

Another way of suspending the prosthesis may be through the use of metamaterials, including auxetic materials. These are materials that contain structure (at macroscopic or microscopic level) that can be 'programmed' to exhibit a bi-stable behaviour: depending on the load applied or temperature, these materials adopt a secondary shape, or perform specific actions. Once example behaviour is where the material has a negative Poisson's ratio, such that it becomes thicker when stretched and thinner when compressed. Other auxetic materials may demonstrate expansion and contraction based on changes in temperature. Such materials can be engineered so that forces applied to them can be transmitted in different direction. For example, the cells can be designed to deform and produce shear when a normal force is applied.

This idea can be applied to a socket or liner construction, with a mesh-type of build where the material is able to react to the elevation of temperatures of the residuum or loading in specific locations (for example due to natural swelling of the residuum). Accordingly, the material may expand and accommodate for the volume fluctuation of the stump, resulting in improved comfort. Alternatively, it can open up pores in the material, to allow ventilation, and get rid of excess sweat.

Another exemplary application of such materials is as follows. It is known that the amputee's residuum shrinks throughout the course of the day. Since the residuum circumference reduces, later in the day the amputees tend to apply more pressure at the distal end of the socket, as the side walls of the socket contribute less to weight bearing. This excess of pressure at the distal end can be transformed by the metamaterials to a programmed area (e.g. side walls, to accommodate for the lost volume and apply additional support to the residuum). This mechanism can be individualised (customised) to the patient. This would require scanning of the residuum shape at various times of the day. Accordingly, the patient can be fitted with a socket/liner that would accommodate for the residuum volume changes that are appropriate to him/her.

It is again to be noted that the sockets presented in Figures 1-6 can be worn without a liner.

The outer fabric material may also include wearable electronics to provide further indication and diagnostic information relating to the residual limb. For example LEDs, displays, etc. may be incorporated to indicate pressure readings, temperature or activity information. Wearable thermal power harvesting electronics and devices, for example using Peltier and Seebeck effect, may also be incorporated into the liner and or socket interface materials. Piezoelectric devices and or inductive power generation materials and devices may also be used to harvest energy from movements at the socket interface. The piezoelectric devices may also be used to augment feedback. For example, sensory information from the prosthesis related to position or loading can be used to feedback vibration or other actuation means.

### Casting techniques

Whichever method is used to "capture" the shape of the residuum or limb in question, more than simply surface topography is required.

This is because the socket needs to be:
- able to transfer the loads of ambulation in a comfortable manner with minimal relative movement between the residuum and the socket (and the prosthesis, prosthetic components, orthosis and orthotic components). It is necessary to avoid as much as possible creating a pseudo-arthrosis which would result in reduced control and loss of energy; and
- be a load/pressure tolerant structure and relieve or off-load pressure sensitive areas, minimising axial, torsional and angular relative motion between the residuum and the socket, yet still allowing muscles to function. The stabilising support portions ("struts") need to be positioned and be able to comfortably stabilise deeper bony structures, loading them comfortably via pressure applied through superficial tissues in an anatomical manner, taking into account muscular attachments, functional muscle groups, nerves and any bony anomalies etc. The struts are therefore not necessarily perfectly flat or straight (as is seen in HiFi) because deeper loaded structures will vary in density, there may be sutures, fracture sites or other bony anatomy to consider.

Therefore whatever method is used to "capture" the residuum or limb in question it is necessary to capture more than simply surface topography. It is also necessary to contain and capture the volumes/areas that are displaced by the stabilisation struts, whilst still allowing muscle groups etc. to function. In the case of flaccid tissue that is not contained or restrained the pressure differential between loaded and unloaded tissues can lead to discomfort and the feeling of increased pressure beneath the stabilisation struts.

Consequently, during the casting or "capture" process it is advised that - especially in the case of flaccid residuums - soft tissues be constrained/loaded with a graded compression distal-proximal (aiding venous return) which will alleviate the pressure differential between areas used for deeper stabilisation and other tissues. Similarly in the finished socket such tissues will require soft tissues to be constrained/loaded with a graded compression distal-proximal whilst still allowing contained tissues/structures to function.

Based on the above considerations, the casting jig should consist of a minimum of 3 stabilising, semi-rigid struts which are sized, positioned and oriented such that they all are equally loaded. The amount the struts indent the residuum during the casting can be controlled at multiple residuum levels along the proximal-distal axis. This can be achieved using, for example, a jig consisting of a series of rings/hoops located one on top of another. Each ring/hoop features at least three threaded inserts. Each insert is attached to one of the struts. During casting, the amputee places the residuum inside the rings/hoops. The struts are subsequently tightened by means of the threaded inserts, so that they make an impression on the amputee's residuum, "shaping" it to the geometry desired for the socket concepts described above. Owing to the fact that there are multiple rings/hoops at multiple levels along the proximal-distal residuum axis, the level of compression/indentation of the struts into the residuum soft tissues can be controlled at multiple points.

The casting apparatus may also consist of instrumentation which measures the stiffness and pressure of the residual limb tissues. This can be achieved, for example, by measuring the pressure and or shear forces at the interface and the displacement of the tissue. Various sensing principles may be used, for example, capacitive based sensor or resistive or a combination of others not limited to these. The data from the tissue analysis captured in the casting rig may also be used to create a solid mechanical model of the residual limb structure. The data then is used for selection of liner and /or socket interface materials to improve comfort and reduce the likelihood of excessive tissue stresses occurring when the socket is worn. The same mechanical model can also be used to optimize the compliance of the socket structures to further enhance comfort.

Once a desired shape is achieved, the residuum shape "capture" is taken.

In addition to the typical methods for "capturing" the residuum shape (e.g., use of plaster of Paris), alternative methods may be possible as well. One way of taking a "capture" of the residuum may be to use textiles with directional stretch vacuumed together. After vacuuming, they will hold shape, thus capturing impression of the residuum as described below. Another method may involve scanning the residuum surface placed in a casting jig using an optical scanner.

### Socket control mechanism

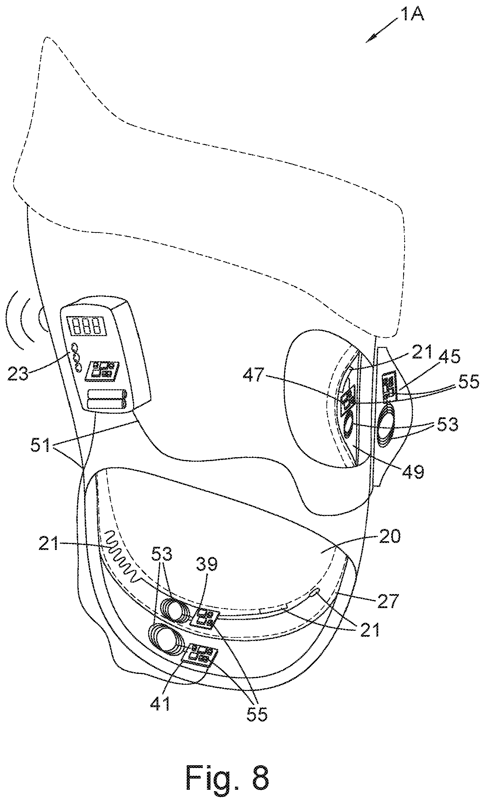

Figure 8 is a schematic representation of a powered 'intelligent' adjustable socket 1A in accordance with an embodiment of this invention. In addition to the features of the socket 1 described with reference to Figures 1A-F and 2-7, the socket 1A of Figure 8 further includes one or more:
- sensors;
- actuators;
- heaters/coolers;
- communications/display modules;
- power modules; and
- power harvesting devices
as is described below in greater detail.

The function of the powered socket 1A is to adjust the socket 1A to provide one or more of:
- better comfort for the user;
- better control of the socket;
- ensure a reliable fit between the socket 1A and the residual limb 20 and/or liner 27 combination, in order to maintain any reduced pressure/vacuum within the socket 1A;
- a self-fitting socket 1A; and
- aid automatic, or semi-automatic alignment.

The socket 1A includes sensors 21 both within the socket 1A and in a liner 27 to be worn by the amputee. The sensors 21 may sense one or more of:
- temperature (sensors include, but are not limited to: thermocouples, thermistors, thermochromic coating, etc.) to measure, for example, the temperature of the residuum and air surrounding the socket;
- movement and position (sensors include, but are not limited to: accelerometers, gyroscopes, inertial measurement units (IMUs), optical sensors, hall effect sensors, etc.) to measure acceleration and velocity of:
   ∘ the residuum in relation to the socket (to assess this, it may be required to measure hip movement. In such case, a movement/position sensor may be attached to the amputee's hip. This can be achieved with a belt worn around the amputee's waist, including the said movement/position sensors);
   ∘ the socket in relation to the ground; and/or
   ∘ the socket in relation to the shank or other body segments.
- flexion (sensors include, but are not limited to: optical sensors, piezoelectric sensors, resistive flexion sensors) to assess bending/flexion of the socket elements, for example to provide feedback to the actuators or indirectly measure loading that goes through the prosthesis;
- humidity (sensors include, but are not limited to: capacitive sensors, resistive sensors, conductive sensors, etc.) to assess microclimate of the residuum, etc.;
- oxygen levels (sensors include, but are not limited to: optical oximeters, etc.) to assess blood level oxidation, and hence enable assessment of tissue viability status, muscle effort, ischemia, etc.;
- pressure, both positive and negative (sensors include, but are not limited to: capacitive sensors, MEMS, force sensing resistors, optical sensors, pneumatic sensors, strain-gauge-based sensors, piezoelectric, piezochromic coating, etc.) to assess residuum loading, socket fit, comfort, amputee intention, socket alignment, etc.;
- shear/friction (sensors include, but are not limited to: capacitive sensors, MEMS, optical sensors, strain-gauge-based sensors, piezoelectric sensors or piezochromic coating, etc.) to assess residuum loading, socket fit, comfort, amputee intention, socket alignment, etc.;
- bacteria (sensors include, but are not limited to: electrochemical sensors, optical) to measure stump hygiene, etc.;
- load/bending moment sensors (sensors include, but are not limited to: resistive sensors, strain gauges, etc.) to measure loading/moment of force that goes through the prosthesis;
- socket proximity/touch/contact to the residuum (sensors include, but are not limited to: optical, capacitive, LVDT, linear potentiometers, contact switches) to detect contact (or lack of it) of the residuum and socket or residuum and liner, or to determine the distance between the residuum and socket or residuum and liner in case the lack of contact, etc.;
- vibration sensors (sensors include but are not limited to: piezoelectric, accelerometers, gyroscopes) to detect taps on the socket (to give additional control over the prosthesis by the user), gait events, etc.;
- electromyography sensors (sensors include but are not limited to: electrodes placed at the skin surface and as form of implants embedded in the soft tissue) to measure muscular activity through electric signals, hence amputee intention, activity level, etc.; this includes both a configuration with one or many sensors. In case a multitude of sensors is used, the high number of sensors may be used to decompose the electromyographic signals to stimuli from individualised neural paths, and therefore work out control (stimulation) signals;
- mechanomyography sensors (sensors include but are not limited to: sensors placed at the skin surface and as form of implants embedded in the soft tissue) to measure muscular activity through mechanical signals, such as audio/vibration signals, etc., hence amputee intention, activity level, etc.; this includes both a configuration with one or many sensors. In case a multitude of sensors is used, the high number of sensors may be used to decompose the electromyographic signals to stimuli from individualised neural paths, and therefore generate control (stimulation) signals;
- stretch sensor (sensors include but are not limited to: resistive and capacitive textiles, rubbers impregnated with resistive compounds, etc.) to measure the residuum shape and size along the circumference, longitudinal axis and any other axis, hence estimate residuum volume fluctuations, muscle volume changes due to contractions, thus providing feedback on the muscular activity, but also to measure tension on the straps, belts and other socket adjusting elements; and
- GPS to monitor motion/activity of the patient.

Data generated by each sensor 21 is passed to a microprocessor forming part of a control unit 23. The data may be passed directly to the microprocessor or by means of an intermediate processor, which may be part of the sensor 21. Instead of the control unit 23 being part of the socket 1A as shown in Figure 2, the control unit may be external to the socket 1A. In the former case, the control unit 23 may use the data generated by the sensors 21 to operate the socket's actuators, to loosen or tighten the socket 1A or to change the relative position of the panels. Where the control unit is external to the socket 1A, data generated by the sensors 21 can be transmitted to the control unit 23 by a communications module, which may include the intermediate microprocessors. Decisions based on the sensor outputs may be based on a signal from a single sensor or a data fusion algorithm may be employed, which incorporates a number of sensor signals as input. Appropriate calculations and processing of sensor signals to perform decision-making can be performed on any microprocessor involved in the data transfer, or in the cloud, as described below.

The communications modules comprise one or more wired or wireless transmitters or transducers which are mounted within the socket 1A, liner 27 or other part of the lower limb prosthesis system. Wireless communications modules can use any wireless communications protocol, such as Bluetooth (RTM), RF, WiFi (RTM) or near field communications. The communications module may be used to communicate between parts of the socket 1A and with external controllers. The external controllers may be part of a dedicated controller device or may be the microprocessor of a mobile device such as a phone 29, tablet or laptop 31, as shown in Figure 3. The mobile device 29, 31 may run dedicated software which may be in the form of an app. The software receives data from the communications module and uses it to decide if action should be taken at the socket 1A as is described below. The mobile devices 29, 31 may further communicate wirelessly (as shown in Figure 3) or wiredly to other parties, such as a medical centre 35 (where prosthetists are based) or a data centre 37. External devices may additionally include other prosthetic joints, such as an intelligent knee 33.

The communications modules may further comprise storage means for storing data generated by the sensors 21. The storage means may be internal or may be removable storage means, such as an SD card or the like. Where collated data is stored in the storage means it may not be transmitted externally in real time but may be transmitted only at predetermined intervals. By transmitting data at discrete intervals the socket's 1A power consumption is reduced.

Instead of or in addition to the communications modules including a wireless transmitter or transceiver, they may include a wired transmitter or transceiver. The wired transmitter or receiver may include a port for connecting a cable, such as a USB (RTM) or micro USB cable or may include a cable which is hard wired to the socket.

In the embodiment shown in Figure 8, the communications modules includes transceivers for communicating externally of the socket 1A, and the communications modules also includes internal transceivers. In particular, the communications modules includes a near field communications (NFC) transceiver 39 in a liner 27 and a corresponding NFC transceiver 41 in the socket wall 47 of the socket 1A to receive data from sensors 21 within the liner 27. There may additionally be an NFC transceiver 45 mounted on the socket 1A which communicates with an NFC transceiver 47 which is within a gel pad 49 internally of the socket 1A. The transceivers 41, 45 which are attached to the socket 1A are connected via wires 51 to the control unit 23. In other embodiments the transceivers 41, 45 may be connected wirelessly using a wireless communications protocol. In other embodiments the transceivers and control unit may form a single unit. These transceivers 39, 41, 45, 47 also serve the function of transferring power from the socket 1A to some or all of the electrical components in the liner 27 and gel pad 49.

In particular, each of the wireless transceivers 39, 41, 45, 47 comprises a coil 53 which is connected to a transceiver circuit 55. The transceiver circuits 55 generate a modulated current in the coil 53 which produces magnetic induction in the loop antennas of the coil 53 to produce a field which is picked up by the corresponding coil 53. In addition, the transceiver circuit 55 of each transceiver 41, 45 in the socket 1A generates a field from its coil 53 which generates a current in the corresponding coil 53 of the NFC transceivers 39, 47 in the respective liner 27 and gel pad 49. In this manner power is transferred from the socket 1A to the liner 27 and gel pad 49 and either stored in the liner 27 and gel pad 49 or the liner 27 and gel pad 49 are continuously powered via the transceivers 41, 45, 55. Note that in power transfer operation the transceivers 41, 45 of the socket 1A act as power transmitters (and not as power receivers) and the transceivers of the liner 27 and gel pad 49 act as power receivers (and not as power transmitters).

In another embodiment, the NFC transceivers 55 are substituted with wireless power and data transfer modules, which operate on the following principle. Transceiver circuit 55 generates a modulated current in the coil 53, which produces magnetic induction in the loop antennas of the coil 53 to produce a field which is picked up by the corresponding coil 53. The current generated in the corresponding coil 53 in liner 27 or gel pad 49 is subsequently picked up by transceiver 55 and either stored in the liner 27 and gel pad 49 or the liner 27 and gel pad 49 are continuously powered via the transceivers 41, 45. The communication between transceivers 55 in socket 1A and corresponding transceivers in liner 27 or gel pad 49 is achieved through the modulation of frequency in transceiver 55 in socket 1A or impedance in corresponding transceivers in liner 27 or gel pad 49.

In other embodiments the communications functions of the transceivers may be separated from the power transfer function. In other words, where coils are used for power and data delivery between parts of the sockets 1A, the coil is used only to transfer power from a power transmitter side to a power receiver side. Communication between the socket 1A and liner 27 or gel pad 49 can be achieved by means of other wireless communication involving coil antennas (Bluetooth, RF, NFC, ZigBee, WiFi, etc.) or optical communication (e.g. infrared).

In one embodiment, the liner 27 is a pin-lock liner. The transceiver circuit 55 and coil 53 located in the liner 27 are placed on an umbrella of the liner 27. Once the pin-lock liner is donned, the pin-lock mechanism locates the liner in place and secures the suspension of the residuum and the socket. The locating feature of the pin is used for locating the coils 53 in the liner 27 and socket 1A, so that their concentricity is ensured. In such case, the pin of the pin-lock liner is made of polymer material or the design of the coils 52 accounts for the presence of a metallic pin in their centre, so that the resultant inductance of the transceiver coils 55 in mated condition is suitable for the modulation frequency of the transceiver circuit 55.

In another embodiment, the coil 53 electrically connected to the liner 27 is located in an enclosure outside of the liner 27. Nonetheless, the coil remains electrically connected to the liner 27 by means of wires that extend proximally from the liner. Coil 53 in the socket 1A is placed on the outside, at a proximal location. To allow power transfer between coils 53 electrically connected to the liner 27 and socket 1A, the amputee would need to attach the coil 53 electrically connected to the liner and the coil electrically connected to the socket after donning the socket. The method of attachment of the coils may involve magnetic attraction or a mechanical clip mechanism.

In another embodiment, there are multiple coils inside the liner 27. When the socket is donned, the field generated by the coil 53 located in the socket 1A induces current in one of the coils located in the liner 27. Transceiver circuit 55 located in the liner 27 is able to detect in which coil current is being induced and disconnects other coils, receiving power from the coil which is best placed to receive the power.

In another embodiment, there are multiple coils inside socket 1A, and one coil on the liner side. Similar principles as above apply.

In an alternative embodiment, the socket 1A may include a microprocessor as well as one or more communications modules communicating with an external microprocessor. In such a case the socket 1A may operate automatically by default under the operation of the internal microprocessor whilst the external microprocessor may be used to additionally control the socket 1A or override the operation managed by the external microprocessor. For example, where the internal microprocessor determines to tighten or loosen the socket by operating the actuators, the user may be able to reverse this operation for reasons of comfort or control, by means of an app or the like. Alternatively, the user may be able to override the tightening or loosening action my manually intervening with the socket 1A itself, either by a manual override or by operating a switch on a wall 43 of the socket 1A.

Similarly, the socket 1A may communicate with other prosthetic limbs, such as intelligent ankles, knees, data collection modules, sensors, etc. 33 having their own microprocessors, to provide an integrated limb control system. This includes ankles, knees, data collection modules, sensors, etc. located on the contralateral limb of the same amputee, if an amputee is a bilateral. For example, rather than the sensors 21 being used primarily for sensing the state of the residual limb 20, liner 27 and socket 1A, the sensors 21 may be used to provide gait analysis for controlling other limbs. Furthermore, other prosthetic limb elements, such as intelligent ankles, knees, data collection modules, sensors, etc. 33 may send data to the socket, so that it can perform a specific action according to those signals.

Once it is determined that a tightening or loosening action is required, the socket 1A may use one or more of its actuators to effect a tightening or loosening operation. The actuators may be one or more of:
- motors to tighten cords (by means of the reels described above, or the like);
- straps that are pre-tensioned by the amputee (ratchets/Velcro (RTM)), however fine-tuning of the strap tension is actuated by a motor or another linear actuator;
- motors to drive threaded inserts (rotational motion of the insert can adjust the protrusion of the insert into the socket). In one embodiment, the socket may have a rigid outer layer and a flexible inner layer; the threaded insert would go through the rigid outer layer but not through the flexible layer. Adjusting the insert will push the inner flexible layer in or out of the socket, effectively modifying its shape;
- inflatable inserts, such as bladder 61, within the socket 1A;
- hydraulic inserts, such as bladder 61, within the socket 1A;
- actuators made of smart materials such as electroactive polymers (e.g., http://www.tecedmonton.com/polymer-based-muscles-and-actuators/) other materials that change shape and/or their elastic properties when subjected to an electric or magnetic field, including, but not limited to dielectric materials (e.g., https://phys.org/news/2016-07-artificial-muscle-soft-robotics-voltahe.html).
- pneumatic or hydraulic actuation:
   ∘ linear actuators (e.g., http://www.linearmotiontips.com/linear-actuators-pneumatic-or-electric/ with tie rod and rodless);
   ∘ pneumatic/hydraulic muscles (e.g., https://en.wikipedia.org/wiki/Pneumatic_artificial_muscles);
   ∘ pneumatic/hydraulic tubing such as that used in rehabilitation gloves or soft exoskeletons (e.g., https://www.ipi-singapore.org/technology-offers/soft-robotic-gloves-and-socks-hand-and-ankle-rehabilitation).

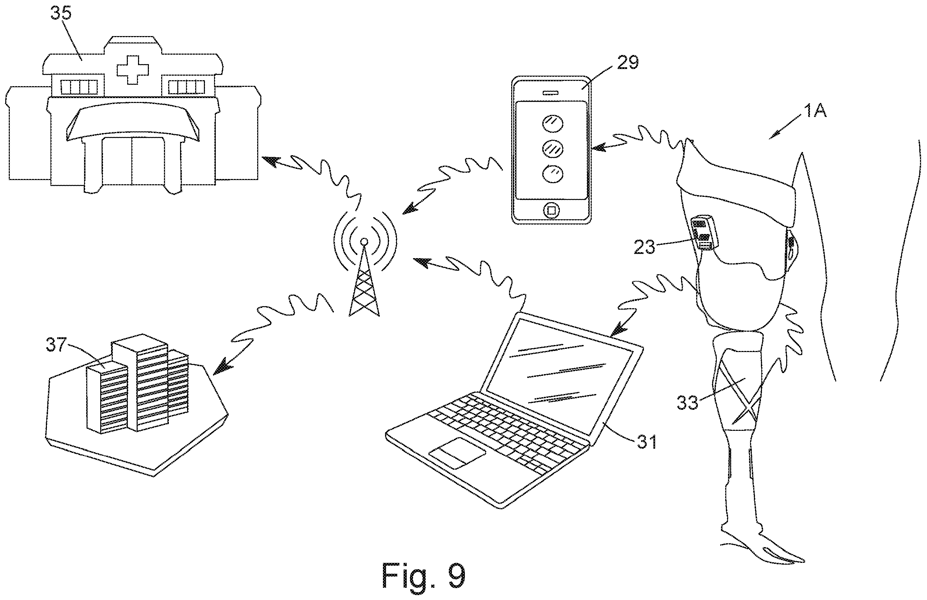

The actuators may achieve socket shape change through pushing portions of socket in or out. For example, they may push entire support portions (e.g., 85, Figure 8), "supracondyles" (Figure 8), adjustable pads (80, 73, 69) or portions of the socket, as illustrated in Figure 9. The rotational and linear motion of an actuator can also be transformed with metamaterials/auxetic materials described above to modify the shape of the socket/liner in the way that is desired or even customised to the specific patient. The actuators may include:
- motors to drive Bowden cables;
- electroactive polymers;
- motors to drive artificial muscles (e.g. made of fishing lines http://lo9.gizmodo.com/scientists-just-created-some-of-the-most-powerful-muscl-1526957560), whereby the actuation technique relies on twist or pull of the line. This includes muscles that are woven into the fabric;
- motorised lace-tightening mechanisms (e.g., http://www.youtube.com/watch?v=XOAGcK9vCqc);
- artificial muscles made of nanotubes, aerogels etc., including materials that exhibit high Poisson's ratio (e.g., http://www.youtube.com/watch?v=xMGXgT0LWUI);
- shape-memory alloys, which are actuated thermally. This allows the socket to accommodate volume changes upon application of heat. For example, the socket made of a shape memory alloy can be 'programmed' to take a specific shape when the residuum begins rubbing against the socket, thus generating heat;
- insets such as 'bean bags'; when subjected to vacuum, they will hold shape. This actuation mechanism allows self-adaptation of the socket to the residuum;
- textiles with directional stretch vacuumed together. In particular, some textiles can be woven in a way that they exhibit directional stretch in a particular direction. Accordingly, an 'actuator' would consist of a few layers of such textiles placed between layers of flexible material that is air-impermeable. The textile layers have to be arranged in a way that at least one of the textile layers is placed so that its stretch direction is different to other layers. When elevated vacuum is applied to such a stack of layers, this material will hold its shape, thus allowing adjustment to any residuum shape;
- inserts with non-Newtonian fluid (e.g. 'silly-putty'). Non-Newtonian fluids are materials that do not follow Newton's law of viscosity. The viscosity of these materials is dependent on the shear rate. Materials such as D3O (RTM) have shear-thickening properties, also called Dilatant, so that as the shear rate increases, the material becomes more liquid. In the case of D3O (RTM), the material is a polyurethane foam with shear-thickening behaviour so that it behaves like a foam (flows) when it is subjected to low shear rates but when this shear rate increases (impact), the molecules lock, absorbing the energy of the impact. An example of application of D3O (RTM) materials is when used in ballet shoes: the molecules inside the material flow freely for comfort when dancing (low shear), but lock together to absorb jarring on landing from jumps. A similar behaviour can be observed on polyurethane foams like Poron (RTM), where the open-cell foam is able to absorb impacts. This material, commonly used in insoles for orthotics. The energy absorption can be easily observed by dropping a ball bearing onto a sample of different materials. Non-energy absorbing materials will make the ball rebound to similar heights to where it was dropped from. However, shock-absorbing materials will dampen the energy and barely rebound. This concept can be applied as a patch or layer inside a socket or liner: the material behaves as a normal socket interface material (i.e. a foam) but when a high impact force is applied (high shear rate), the material will lock, absorbing the energy and dampening the damage on the stump. Such inserts can be entirely passive (i.e., no actuation at all, such materials can be used to absorb energy), or actively actuated (e.g. when placed in a bladder and subjected to an increase in pressure, they will firm up). Non-Newtonian materials can be used also in construction of the socket - for example, if placed along the line 89 in Figure 5 (as a hinge), they can act as a form of stumble prevention mechanism. In particular, during regular walking, a prosthesis 84 hinged about line 89 can allow the support portions 85 to flex. However, when an amputee stumbles, high loading will be applied to the support portions 84. In reaction to that high loading, the hinge will firm up, and thus, support portions will ensure good control over the prosthesis, which is required during stumble recovery.
- motors to drive pyramid alignment interfaces and thus provide the angular adjustments to the socket alignment. This is important during alignment assessment, however the socket can align itself according to the sensor feedback, irrespectively of alignment performed by prosthetist.

Data generated by the sensors 21 can be used to identify one or more of the following conditions and the following actions can be taken to address each condition:

### Self-learning system

The socket may send data to a cloud storage system 37 for storage and processing to optimise future designs, as well as to improve performance of the existing devices. The data collected by the socket system may include (in raw or processed form):
- type of prosthesis elements included in the prostheses, their revisions, including software revisions, identifiers, settings of their actuators, including knee and ankle valve positions, socket actuators states, memory states etc.;
- data from all sensors, including sensors located in the sockets, liner, pads from other prosthetic limb elements, such as intelligent ankles, knees, data collection modules, sensors, etc. 33.;
- socket comfort score and other feedback from the amputee, for example collected with an app; and
- instances when an amputee manually overrides the settings of the socket, which may suggest that the socket settings selected by the embedded algorithm are not suitable.

These transmissions may include continuous streams of data, bursts of data, counts of instances a specific event occurred, timing of those events, etc.. In addition to the data transmitted by the socket, during fitting or appointments, prosthetists may also be able to log the interventions they performed, as well as patient's particulars, including the patient's as age, weight, residuum shape, residuum status, etc., but also 3D scans of the stump and socket, X-ray images, MRI outputs etc..

When the stored data is processed, it employs cloud-based computing systems (e.g. IBM Watson (RTM) computing scheme), however parts of the computing can be done by the microprocessor on the prosthesis or microprocessor in the telephone/mobile device. A range of different processing techniques may be used, including, but not limited to:
- statistical analyses, including regression analyses;
- machine learning and deep learning methods, artificial neural networks, Boltzmann machine, etc.;
- optimisation algorithms;
- image processing and recognition techniques; and
- pattern processing and recognition techniques (for example movement pattern recognition);

In another embodiment the above-mentioned calculations may be performed in part or in full on any microcontroller located in the prosthesis.

Through identifying dependencies and correlations between different pieces of collected data, the above-mentioned techniques will aim to achieve the following:
- predict health problems;
- improve algorithms in the microprocessors in the prosthetic knees, ankles, sockets etc. including modification of existing parameters, coefficients or thresholds of existing algorithms or aid development of new algorithms;
- predict amputee's intention;
- identify the needs of the amputees (and hence feed to new prosthetic designs);
- detect activity types and levels/intensities;
- manage residuum microclimate;
- improve amputee's comfort and control over the prosthesis; and
- detect amputee comfort, identify pistoning, rubbing, elevated heat, sweating and identify appropriate actions to act accordingly.

The outcomes of the processing may be used:
- in scientific reporting as evidence for the prosthetic outcomes;
- implementation of new designs; and
- improvements to existing designs, in particular improving the actuation of the socket and its adaptability to amputee activities,
eventually, benefitting from the data collected from many patients, but prioritising data from the patient wearing the individual preferences of the patient, the socket will become individualised, and will be able to "grow" and adapt to the patient, hence leading to reduced need for appointments with prosthetists, etc..

### Assessing Risk of Health Problems

Factors such as the loads, temperature, humidity, bacteria or other microclimate indicators at the residual limb 20 can be assessed, for the purpose of checking that these factors do not exceed predetermined levels or operate within certain limits. If these factors exceed the predetermined levels there may be a risk of pressure ulcers (PU) or other health problems.

In response to identification of a health risk one or more of the following steps can be taken:
- inform the amputee by beeping, light flashing, vibration (additional hardware components may be required for this);
- send data to the user's mobile phone 29 to alert the amputee;
- send data to a clinic 35 for reference for a prosthetist;
- send data to a cloud storage and processing system 37, as described in previous section;
- operate heating (or cooling) within the socket 1A; and
- store data on an SD card or other memory or send data to a PC or mobile device for scientific evaluation.

### Assessing the amputee's comfort

Data from the sensors 21 can be used to assess how comfortable the stump 20 is within the socket 1A by measuring:
- pressure, shear: when interface loading (in direct form such as current pressure/shear reading, or indirect form, such as pressure/shear peak, pressure/shear time integral, pressure/shear peak duration, mean pressure/shear over predetermined time period or gait cycle(s)) crosses a predetermined threshold, is within a predetermined range or exhibits a specific spatial/temporal pattern, may be indicative of discomfort (prosthesis causing pain, also potentially posing risk of health problems);
- volume and shape: change in residuum volume and/or shape may be indicative of change of fit;
- socket proximity: the detection of lack of contact between the socket and the residuum may be indicative of the change of the residuum shape and hence poor fit; the bigger the gap, the greater need for correction;
- movement: when the movement of the residuum inside the socket exceeds a predetermined threshold, is within a predetermined range or exhibits a specific spatial/temporal pattern, it may be indicative of lost/excessive suspension;
- temperature and moisture: when the temperature and/or humidity of the residuum exceeds a predetermined threshold, is within a predetermined range or exhibits a specific spatial/temporal pattern, it may be indicative of excessive heat, which may be a result of stump rubbing against the socket, excessive work conducted by the limb, infection, etc..

In response to an assessment of a lack of comfort, signals can be sent to other prosthetic components, particularly to the socket's actuators as well as to the ankle and knee 33 to optimise comfort. This may be achieved in one or many of the following ways:
- the prosthetic socket may change its shape if an excess of pressure/shear is detected. Appropriate action to relieve the affected location is performed, or an amputee is advised to do so manually via vibration/sound/visual signals. The predetermined thresholds/regions can be readily altered using self-learning methods described above, and feedback from the amputee;
- the prosthetic socket may change its shape in attempt to maintain a predetermined spatial or temporal profile/pattern of pressure and shear. The profile may be individualised to the patient through a calibration conducted when the socket is fitted and considered comfortable. From then on, the socket will make adjustment to ensure that the pressure/shear profile similar to that predetermined one is maintained. The predetermined thresholds/regions can be readily altered using self-learning methods described above, and feedback from the amputee;
- the prosthetic socket may change its shape to prevent discomfort due to the redistribution of the loading caused by a change of shape. Accordingly, the prosthesis may perform corrective action, to accommodate the change in the shape. The goal is to make adjustment to the socket shape, so that at all times it matches the stump;
- prosthetic socket changes its shape based on the information on the residuum movement inside the socket. Accordingly, the socket changes its shape by providing a more tight fit when the movement is too excessive and relaxing the fit when the not enough movement is present; and
- prosthetic socket changes its shape as well as performs actions to cool the residuum down or evacuate sweat away from the residuum surface when excess of heat/moisture is detected.

In one embodiment, the actuation of the prosthetic socket is performed in the swing phase of gait. This is because the prosthesis is not in contact with the ground and hence the socked is subjected to minimal loading. Therefore, the actuators will not need to 'fight' against the loading arising from gait and hence the socket actuation can be achieved at lower energy/power expenditure.

### Identification of the relevant amputee activity

The signals from the sensors, including, load and motions sensors, can also be used to determine amputee activity. This can be used for collection of data on outcome measures as well as justification of the amputee's needs, for reimbursement purposes. Additionally, determination of the activity can be used in other decision-making processes. For example, depending on the activity performed by the amputee, thresholds, permitted ranges of operation, spatial or temporal patterns that are used to perform any functionality described in this patent may vary.

### The Intention of the Amputee to accelerate or decelerate

The sensors 21 can be used to assess the intended walking behaviour of the amputee, e.g., if the amputee wants to accelerate, decelerate. This can be detected, for example, through sensing sagittal (antero-posterior) shear from a sensor located at the distal end of the socket or two pressure sensors - one located at the anterior distal and one at posterior distal aspect of the socket. When an amputee intends to decelerate, one or more of the following can indicate this intention:
- excess of pressure measured at the anterior distal pressure sensor during stance phase of gait;
- reduction of pressure measured at the posterior distal sensor during stance phase of gait;
- pronounced anterior-posterior shear (posterior direction when measured at the socket) at distal end during the first half of the gait cycle; and
- reduced anterior-posterior shear (anterior direction when measured at the socket) at distal end during the second half of the gait cycle.

In contrast, when the amputee wants to accelerate one or more of the following can indicate this intention:
- reduction of pressure measured at the anterior distal pressure sensor during stance phase of gait;
- excess of pressure measured at the posterior distal sensor during stance phase of gait;
- reduced anterior-posterior shear (posterior direction when measured at the socket) at distal end during the first half of the gait cycle; and
- pronounced peak of the anterior-posterior shear (anterior direction when measured at the socket) at distal end during the second half of the gait cycle.

The predetermined thresholds, signal ranges, spatial or temporal patterns that permit identification of amputee intention might vary depending on activity. This may permit, for instance, ability to slow down and speed up walking on slopes (both up and down).

Other sensors may also be utilised to perform detection of the amputee's intention. This includes electro- and mechanomyographic sensors, or stretch sensors, all of which can be used to monitor muscle contractures. Signals from appropriate muscle contractures can be used as control signals, whereby the amputee can send commands to the prosthesis in a similar way the signals are sent to upper limb prostheses, as is known in the art.

In response to identification of the amputee's intention, one or more of the following steps can be taken:
- actuate the socket in a manner which ensures an adequate level of control and comfort for a given intended action, or that improves power efficiency;
- send data to other prosthetic components, e.g., one or more of the ankle, knee 33 and socket 1A, to change stiffness/or alter power delivery (if powered components are used); and
- store data on an SD card or other memory or send data to a PC or mobile device for scientific evaluation.

### Detecting when to release the knee

Factors such as the loads, pressure, shear, at the residual limb 20 as well as kinematics, e.g., motion of the residuum in the socket, can be assessed for the purpose of checking that these factors do not exceed predetermined levels, or operate within certain limits. If these factors exceed the predetermined levels or operate within certain limits, this might indicate the timing of the knee release. In response to identification of such event a signal might be sent to a knee to release from stance phase into swing.

Other sensors may also be utilised to perform detection of the amputee's intention. This includes electro- and mechanomyographic sensors, or stretch sensors, all of which could be used to monitor muscle contractures. Signals from appropriate muscle contractures can be used as control signals, whereby the amputee may send commands to the prosthesis in a similar way the signals are sent to upper limb prostheses, as known in the art.

### Detecting seated position and/or intention to seat/stand

Factors such as motion and/or load may be used to determine that the amputee is seated. In such a case, the socket would open up to allow venous return. In contrary, when the intention to stand up by the amputee is detected, the socket re-adjust to provide additional support. During both sit-to-stand and stand-to-sit, signals can also be sent to other prosthesis components such as ankles and knees, to determine their flexural properties. For example, a foot setting would change to high resistance to plantarflexion, low resistance to dorsiflexion (during both sit-to-stand and stand-to-sit). A knee setting would change to high resistance in flexion, low resistance in extension.

### Tracking of Stump Motion

The sensors 21 can be used to monitor and track relative stump 20/socket 1A motion inside the socket 1A (through movement and position sensors), so as to assess if pistoning or angular coupling of the residual limb 20 is taking place within the socket 1A. (Coupling refers to relative movement between the socket and the residuum. There is axial coupling (or pistoning) when the residuum moves along the axis of the socket, but also there exists angular coupling, which refers to angular motion of the residuum and socket.) Among others, this will permit determining risk of blisters/stump health issues, and assess the quality of the socket suspension. Achieving adequate suspension is required to ensure that the amputee is confident in putting the right loading through the prosthesis. Inadequate/asymmetric loading put through the prosthesis may lead to health problems such as back pain.

In response to tracking the stump's 20 motion, one or more of the following steps can be taken:
- Adjust the prosthesis by tightening the adjustable pads, and hence stabilising the bony remnant;
- Store data on an SD card or other memory or send data to a PC or mobile device for scientific evaluation;
- Alert the amputee that an excess of coupling occurring;
- Send data to the cloud storage and processing system for processing; and
- Send data to other prosthetic components, in particular adaptive socket to change shape and ensure an adequate level of control over the prosthesis.

### Stump position within the socket

The sensors 21 can be used to assess if the socket 1A is in the same place as it was when it was fitted, i.e., if it has been subject to any rotation. This can be done by comparing the sensed pressure profile with that recorded when the socket 1A was initially fitted, or at the beginning of the day.

If it has been determined that the stump 20 has moved within the socket 1A one or more of the following steps can be taken:
- Store data on an SD card or other memory or send data to a PC or mobile device for scientific evaluation;
- Alert the amputee that the position of the residuum in the socket is inappropriate;
- Send data to the cloud storage and processing system for processing; and
- Send data to other prosthetic components, in particular adaptive socket to change shape and adjust to ensure an appropriate loading spatial-temporal profile.

### Detecting a stumble or trip

The sensors 21 can be used to detect a risk or presence of a stumble or trip by sensing unexpectedly high pressures at the stump 20, using pressure sensors or vibration sensors, despite that the limb 20 is in swing phase.

In response to a determination of a potential or actual stumble/trip the socket 1A can send data to other prosthetic components, e.g., one or more of the ankle, knee 33 and socket, to trigger stumble mode operation. This information would temporarily firm up the prosthetic knees, ankles and sockets, thereby ensuring improved control over the prosthesis by the amputee as well as reliable means of loadbearing subsequently to the tripping or slipping event.

It is also possible, that other prosthetic components may detect stumble and inform the socket about such a potential event. In such a case, the socket would firm up to ensure the amputee can control the socket.

Additionally, the following actions can be taken:
- Store data on an SD card or other memory or send data to a PC or mobile device for scientific evaluation; and
- Send data to the cloud storage and processing system for processing.

### Assessing the level of vacuum in the socket

Additionally, the sensors 21 can be used to assess the level of vacuum in the socket 1A. If an insufficient vacuum is detected, data is sent to the other prosthetic components, in particular to the socket 1A, to improve the seal, or to a vacuum pump to improve the suspension.

Additionally, the following actions can be taken:
- Store data on an SD card or other memory or send data to a PC or mobile device for scientific evaluation;
- Send data to a PC or mobile device, to alert the amputee about the poor seal, or use an alternative method of indication of poor seal, such as on-socket display, sensory feedback with vibrators, sound signals, etc.; and
- Send data to the cloud storage and processing system for processing.

### Detecting excess sweat

The sensors 21 can additionally or alternatively be used to assess the amount of moisture in the socket 1A which may denote a build-up of excess sweat. This can be remedied by activating cooling elements and/or actuating the liner's and socket's smart materials, that open up pores.

### Debugging

Data from the sensors may also be stored internally for debugging purposes. The data may be downloaded by the prosthetist and used as an outcome measure. Furthermore, the data from all the sensors (both live stream and historic) can be accessed remotely either by the prosthetist or by the prosthesis manufacturer/engineers/service centre. The data can be used to remotely alter the properties/settings of the prosthesis (this includes changes of predetermined thresholds or allowed ranges, but also software on the devices), and collection of data for debugging of the prosthesis performance off-line.

### Bilateral prosthetic devices

Signals from the sensors, such as load (pressure, shear), kinematics (motion sensors), activity/mode etc. can be exchanged between bilateral limbs to identify:
- Double support. This data can be collected for measuring intervention outcomes. Knowing the loading on the contralateral limb may also be used to aid stumble and knee release timing;
- Kinematic and kinetic variables can be used to assess symmetry of gait. According to the identification of non-symmetrical gait of the patient, the prosthetic components can be altered (for example, damping/resistance of the components can be changed to control velocities, range of motion, loading, etc.) to ensure a more symmetrical gait; and
- What mode is the contralateral prosthetic component is in to make sure that the other limb is in the same mode.

### Sensory feedback

The sensor signals can be used to provide a sensory feedback to the amputee. More specifically, many amputees (particularly above knee amputees) suffer from the lack of sensation such as:
- where the limb is;
- what loading goes through it;
- where and how hard it touches the ground and other objects; and
- where and how high the temperature/humidity of the surrounding is, etc..

Having that sensation allows able bodies have a good control over the limb. To provide some form of sensory feedback, signals from sensors such as:
- knee and ankle angle;
- force (pressure/shear/load) sensors loading going through the prosthesis; and
- temperature sensors,
can be converted to stimuli fed back to the amputee. The sensation can be achieved by:
- vibrator(s);
- heating element(s); and
- pins extending or contracting.

The vibrators/pins/heating elements may be placed in multiple positions in the socket/liner. The intensity, number of, or spatial/temporal pattern of the provided stimuli may correspond to the strength of measured variable. The stimuli may also vary depending on whether the variable crosses a predetermined threshold, is within a predetermined range or when a specific spatial/temporal pattern is detected.

Furthermore, additional stimuli may be used to provide a sensory feedback. Purpose-made feedback devices attaching to the body by means of adhesion or straps can be used in case of patients with tactile impairments at the stump. The devices can contain vibrators/pins/heating elements. Similarly, vibrators/pins/heating devices can be placed on a smart watch. Furthermore, the feedback may be provided through augmented reality, whereby the patient wears goggles or glasses and an image of a prosthetic leg, or another visual aid is superimposed on the view of the amputee and is used to indicate the strength of the measured variable.

### Assisting fitting and alignment

Signals from the pressure/shear, contact, sensors may be used to provide feedback to the prosthetist regarding the socket's alignment. In one embodiment, the prosthesis may inform the prosthetist about the magnitude of loading applied to the posterior/anterior or medial/lateral sides of the socket (both during standing, gait or other activities). If that loading exceeds a predetermined threshold, is within a certain range or exhibits certain spatial or temporal pattern, this can trigger an indication to the prosthetists. This can be in form of sound (beeps) or visual (with LEDs of the prosthesis, display on the prosthesis or on a PC/mobile/smart watch app). Alternatively, the signals may also be used as an input to the automated/motorised pyramid alignment interface, which will perform appropriate adjustments to the alignment in automated manner.

For instance, when during standing the socket sensors suggest one or more of the following:
- excessive pressure at the distal-lateral region;
- excessive pressure at the proximal-medial region; and
- lack of contact at the lateral supracondyle (in the case of transtibial amputees) or proximal-lateral region of the socket,
the alignment of the prosthesis can be altered (either by the prosthetist or by automated/motorised pyramid alignment interface) to provide adduction of the socket and/or medial translation of the socket.

When during standing the socket sensors suggest one or more of the following:
- excessive pressure at the distal-medial region;
- excessive pressure at the proximal-lateral region; and
- lack of contact at the medial supracondyle (in the case of transtibial amputees) or proximal-medial region of the socket,
the alignment of the prosthesis can be altered (either by the prosthetist or by automated/motorised pyramid alignment interface) to provide abduction of the socket and/or lateral translation of the socket.

When during standing the socket sensors suggest the following:
- excessive pressure at the patella-tendon region;
- excessive pressure at the posterior-distal region; and
- and abnormal flexion,
the alignment of the prosthesis can be altered (either by the prosthetist or by automated/motorised pyramid alignment interface) to provide flexion of the socket and/or posterior translation of the socket.

When during standing the socket sensors suggest the following:
- excessive pressure at the patella-tendon region;
- excessive pressure at the posterior-distal region;
- hyperextension of the knee
the alignment of the prosthesis can be altered (either by the prosthetist or by automated/motorised pyramid alignment interface) to provide extension of the socket and/or anterior translation of the socket.

When during standing the socket sensors suggest the following:
- excessive pressure at the popliteal fossa region;
- excessive pressure at the anterior-distal region;
- knee flexion shows no abnormalities,
the alignment of the prosthesis can be altered (either by the prosthetist or by automated/motorised pyramid alignment interface) to provide posterior translation of the socket.

When during standing the socket sensors suggest the following:
- excessive pressure at the patellar tendon region;
- excessive pressure at the posterior-distal region; and
- knee flexion shows no abnormalities,
the alignment of the prosthesis should be altered (either by the prosthetist or by automated/motorised pyramid alignment interface) to provide anterior translation of the socket.

Further to the display of the loading using the app on the mobile device, in another embodiment, the sensor signals can be visualised using augmented reality technology. In such embodiment, the prosthesis user or a prosthetist films the prosthesis using a camera in a mobile device. The device displays in real-time the filmed image but additionally superimposes signal from the sensors (for example, loading information may be displayed in form of vectors, pressure distribution can be displayed as colour maps overlaid over the socket, traffic light indication of high/medium/low level of loading, using red/amber/green colour scheme etc.).

In order to overlay the vectors/maps/other displays over a filmed image of the prosthesis, the mobile device needs to be able to 'recognise' the prosthesis (work out where the prosthesis is). This can be achieved with image processing algorithms allowing for pattern recognition, or using sources of light, such as LEDs (either visible or non-visible spectrum, e.g. infrared) placed on the socket/other prosthetic components. Identification of these LEDs will permit identification of key landmarks on the prosthesis and hence 'recognition' of the prosthesis. The LEDs can either produce continuous beam of light or can flash, each with a specific code, which permits identification of the specific landmarks

Similarly, the augmented reality can be displayed using special goggles (e.g. Google Glass (RTM), or similar). Alternatively, 3D holographic projection technology may be employed to produce similar effect, i.e., overlay of certain variables (vectors, pressure maps, traffic light etc.) over the prosthesis. It is envisaged that this will remove the need for laser/optical methods for postural alignment.

### Collecting outcome measures

Data may be collected by means of one or more of:
- SD card or alternative means of storage in the prosthesis;
- On a mobile device or a PC/laptop; and
- In a cloud storage space.

The collected data may include one or more of:
- Data from the sensors;
- Amputee feedback (using an app/online service), such as the previously mentioned socket comfort score as well as actions (manual adjustments to the socket);
- The instances when the amputee manually overwrites the predetermined settings of the prosthesis, including ankle settings, knee settings and socket settings; and
- Any adjustments to the prosthesis, including ankle settings, knee settings and socket settings that the amputee makes.

The data can be used to monitor the performance of the prosthetic outcome, it may be used to determine amputee activity level, as well as evaluate prosthetic components.

In order to power the sensors 1A, actuators 17P, 17D, control unit 23 and other electrical components in the socket 1A the socket 1A further includes an electrical power storage device, such as a battery, and means for charging the storage device, as described above.

The primary functions of the energy receiver are to receive power from the associated external energy transmitter and to manage the received power (either powering its components only when RF energy available, or charging a battery and feeding components from the battery when RF power is unavailable). Additionally, the energy receiver may incorporate the communications module described above, so that it can communicate with the external energy transceiver.

The energy transceiver is placed inside or outside the socket and its primary functions is to send power to the energy receiver. If the energy receiver also sends data to the energy transceiver then the energy transceiver additionally receives data from the energy receiver and may transmit data/commands to energy receiver.

Data from the sensors 21 in the liner/pad/mat and sent to energy transmitter have to be either:
- collected by the energy transmitter or adjacent electronics connected to it through wireless or wired link and saved to SD card or other memory;
- processed to form data usable by other prosthetic components (e.g., the data may be used to detect certain features of gait and used to trigger different modes in the connected ankle/knee 33, etc.) or other medical devices worn by the amputee and sent to those devices;
- sent to one or more of the external devices (e.g. mobile phone 29, laptop 31, etc.);
- External components/sensors can also send information to the liner/pad to e.g. put heating on within the socket 1A; and
- External components/sensor can also send information to the adaptive socket to e.g. reduce socket tightness when seated.

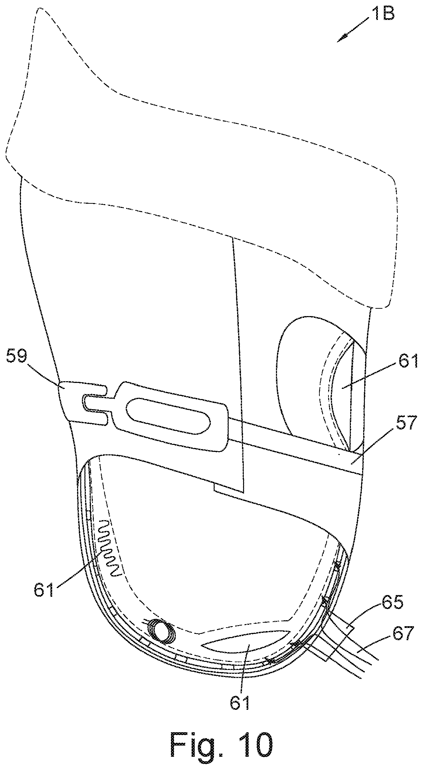

Figure 10 is a schematic representation of an intelligent socket 1B having an alternative configuration to the socket 1A of Figure 2. The socket 1B of Figure 4 has a number of features which are also present in the socket 1A of Figure 8 and are denoted accordingly. The person skilled in the art will readily understand that features of the two embodiments are interchangeable where they are not contradictory.

The socket 1C of Figure 4 does not use motors to tighten cords 15D, 15P as shown in Figure 1, but uses a ski-boot type adjustable buckle 57 and buckle hook 59 to hold the socket 1C around the residuum. In addition, the socket 1C includes bladders 61 whose volume can be changed to compensate for changes in the shape of the residuum 20. The bladders 61 may be pneumatic, hydraulic or magnetic, as is known in the art.

The socket 1C further includes means for cooling/heating the residuum 20 in the form of a thermoelectric element 63 using the Peltier effect. In addition a fan 65 can be incorporated into the socket 1C in communication with channels within the socket 1C and an extraction port 67 through which moisture and sweat can be withdrawn from the socket 1C. In addition, the thermoelectric element 63 can be used to harvest energy from the socket, to charge a battery, which is used as a source of power for the electronic system. Other than the thermoelectric element, the energy can be harvested for example using Stirling engines. Furthermore, energy harvesting may also be achieved with piezoelectric, materials (harvesting of strain, vibration), electromagnetic and electrostatic inertial generators, etc.. Various modifications will be apparent to those in the art and it is desired to include all such modifications as fall within the scope of the accompanying claims.

The scope of the invention is solely defined by the appended claims.

## Claims

1. A prosthetic limb socket (1) comprising:
a rigid support member (3) comprising a distal base portion (5), a first cantilevered support portion (7) extending around a majority of a circumference of the socket (1) and a second cantilevered support portion (9) opposite the first cantilevered support portion (7) and defining a pair of windows (11A, 11P) between lateral edges (7E, 9E) of the first (7) and second (9) cantilevered support portions;
first (13A) and second (13P) adjustable panels, each panel being disposed in one of the pair of windows (11A, 11P) between the first and second cantilevered support portions (7, 9); and
means for adjusting a radial position of the panels (13A, 13P) relative to the support portions (7, 9),
wherein the panels (13A, 13P) are held in position between the cantilevered support portions (7, 9) by the means for adjusting.

2. A socket as claimed in claim 1, wherein the socket (1) is a transfemoral or knee disarticulation socket for a transfemoral or knee disarticulation residual limb respectively.

3. A socket as claimed in claim 1 or 2, wherein:
the first cantilevered support portion (7) extends around a medial portion of the circumference of the socket;
the first panel (13A) extends along an anterior lateral portion of the socket;
the second panel (13P) extends along a posterior lateral portion of the socket; and
the second cantilevered support portion (9) extends along a lateral portion of the socket.

4. A socket as claimed in claim 1, 2 or 3, wherein the first (13A) and second (13P) panels extend proximally beyond the second cantilevered support portion (9).

5. A socket as claimed in any one of the preceding claims, wherein the panels (13A, 13P) comprise a rigid or semi-rigid outer shell (13AS, 13PS) and a resiliently compressible inner portion.

6. A socket as claimed in claim 5, wherein the inner portion comprises a strip of ethylene-vinyl acetate.

7. A socket as claimed in any one of the preceding claims, wherein in combination the cantilevered support portions (7, 9) and the panels (13A, 13P) extend around the circumference of the socket.

8. A socket as claimed in any one of the preceding claims, wherein the means for adjusting a radial position of the panels (13A, 13P) relative to the support portions (7, 9) comprises a cord or cords (15D, 15P) traversing the support portions and the panels.

9. A socket as claimed in claim 8, wherein the cord is or the cords (15D, 15P) are disposed on an outer surface of the cantilevered support portions (7, 9) or embedded within an outer shell of the cantilevered support portions (7, 9).

10. A socket as claimed in claim 8 or 9, wherein the cords (15D, 15P) comprise a first cord (15D) and a second cord (15P).

11. A socket as claimed in claim 8, 9 or 10, further comprising means for drawing the cord or cords (15D, 15P) radially inwards

12. A socket as claimed in claim 11, wherein the means for drawing the cord or cords (15D, 15P) radially inwards comprises a ratchet mechanism.

13. A socket as claimed in claim 12 when dependent on claim 10, wherein the ratchet mechanism comprises first (17P) and second (17D) ratchet reels, each ratchet reel (17P, 17D) drawing in one of the first and a second cords (15D, 15P), the first and second cords (15D, 15P) being spaced distally and proximally of each other along the cantilevered support portions (7, 9).

14. A socket as claimed in claim 11, 12 or 13, wherein the means for drawing the cord radially inwards comprises a motor.

15. A socket (1) as claimed in any one of the preceding claims and further comprising:
a plurality of sensors (21) within the support member; and
means for adapting one or more of:
an interior volume of the support member;
a temperature within the support member;
oxygen levels within the support member; and
a pressure within the support member, and
communications modules for communicating between the sensors (21) and the means for adapting.

## Patentansprüche

1. Schaft für eine Gliedmaßenprothese (1), umfassend:
ein starres Stützelement (3), das ein distales Basisteil (5), ein erstes freitragendes Stützteil (7),
das um den Großteil eines Umfangs des Schafts (1) herum verläuft, und ein zweites freitragendes Stützteil (9) umfasst, das dem ersten freitragenden Stützteil (7) gegenüberliegt und ein Paar von Fenstern (11A, 11P) zwischen den Seitenrändern (7E, 9E) des ersten und des zweiten freitragenden Stützteils (7) bzw. (9) definiert;
eine erste und eine zweite verstellbare Platte (13A) bzw. (13P), wobei jede Platte in einem der beiden Fenster (11A, 11P) zwischen dem ersten und dem zweiten freitragenden Stützteil (7, 9) angeordnet ist; und
ein Mittel zum Einstellen einer radialen Position der Platten (13A, 13P) in Bezug auf die Stützteile (7, 9), wobei das Einstellmittel die Platten (13A, 13P) in ihrer Position zwischen den freitragenden Stützteilen (7, 9) hält.

2. Schaft nach Anspruch 1, wobei es sich bei dem Schaft (1) um einen Oberschenkel- oder Knieexartikulationsschaft für einen Oberschenkel- bzw. Knieexartikulationsstumpf handelt.

3. Schaft nach Anspruch 1 oder 2, bei dem
das erste freitragende Stützteil (7) um einen mittleren Abschnitt des Schaftumfangs herum verläuft;
die erste Platte (13A) an einem vorderen seitlichen Abschnitt des Schaftes entlang verläuft;
die zweite Platte (13P) an einem hinteren seitlichen Abschnitt des Schaftes entlang verläuft; und
das zweite freitragende Stützteil (9) an einem seitlichen Abschnitt des Schaftes entlang verläuft.

4. Schaft nach Anspruch 1, 2 oder 3, bei dem sich die erste und die zweite Platte (13A) bzw. (13P) in proximaler Richtung über das zweite freitragende Stützteil (9) hinaus erstrecken.

5. Schaft nach einem der vorhergehenden Ansprüche, bei dem die Platten (13A, 13P) eine starre oder halbstarre Außenhülle (13AS, 13PS) und einen elastisch komprimierbaren inneren Teil aufweisen.

6. Schaft nach Anspruch 5, bei dem der innere Teil einen Streifen aus Ethylen-Vinylacetat umfasst.

7. Schaft nach einem der vorhergehenden Ansprüche, bei dem sich die freitragenden Stützteile (7, 9) und die Platten (13A, 13P) zusammen um den Umfang des Schafts herum erstrecken.

8. Schaft nach einem der vorhergehenden Ansprüche, bei dem das Mittel zum Einstellen einer radialen Position der Platten (13A, 13P) in Bezug auf die Stützteile (7, 9) eine Schnur bzw. mehrere Schnüre (15D, 15P) umfasst, die durch die Stützteile und die Platten hindurch verläuft bzw. verlaufen.

9. Schaft nach Anspruch 8, bei dem die eine Schnur bzw. die Schnüre (15D, 15P) auf einer Außenfläche der freitragenden Stützteile (7, 9) angeordnet oder in eine Außenhülle der freitragenden Stützteile (7, 9) eingebettet ist bzw. sind.

10. Schaft nach Anspruch 8 oder 9, bei dem die Schnüre (15D, 15P) eine erste Schnur (15D) und eine zweite Schnur (15P) umfassen.

11. Schaft nach Anspruch 8, 9 oder 10, des Weiteren umfassend Mittel zum Einziehen der Schnur bzw. Schnüre (15D, 15P) radial nach innen.

12. Schaft nach Anspruch 11, bei dem das Mittel zum Einziehen der Schnur bzw. Schnüre (15D, 15P) radial nach innen einen Ratschenmechanismus umfasst.

13. Schaft nach Anspruch 12, soweit abhängig von Anspruch 10, bei dem der Ratschenmechanismus ein erstes Ratschenrad (17P) und ein zweites Ratschenrad (17D) umfasst, wobei jedes Ratschenrad (17P, 17D) eine Schnur der ersten und der zweiten Schnur (15D, 15P) einzieht, wobei die erste und die zweite Schnur (15D, 15P) an den freitragenden Stützteile (7, 9) entlang distal und proximal voneinander beabstandet sind.

14. Schaft nach Anspruch 11, 12 oder 13, bei dem das Mittel zum Einziehen der Schnur radial nach innen einen Motor umfasst.

15. Schaft (1) nach einem der vorhergehenden Ansprüche und des Weiteren Folgendes umfassend:
eine Vielzahl von Sensoren (21) im Stützelement; und
Mittel zum Anpassen eines oder mehrerer folgender Werte:
Innenvolumen des Stützelements;
Temperatur im Inneren des Stützelements;
Sauerstoffwerte im Inneren des Stützelements; und
Druck im Inneren des Stützelements, sowie
Kommunikationsmodule für die Kommunikation zwischen den Sensoren (21) und den Mitteln zum Anpassen.

## Revendications

1. Emboîture de membre artificiel (1) comprenant :
un élément de support rigide (3) comprenant une partie base distale (5), une première partie de support en porte-à-faux (7) s'étendant autour d'une majorité d'une circonférence de l'emboîture (1) et une seconde partie de support en porte-à-faux (9) opposée à la première partie de support en porte-à-faux (7) et définissant une paire de fenêtres (11A, 11P) entre des bords latéraux (7E, 9E) des première (7) et seconde (9) parties de support en porte-à-faux ;
des premier (13A) et second (13P) panneaux réglables, chaque panneau étant disposé dans l'une de la paire de fenêtres (11A, 11P) entre les première et seconde parties de support en porte-à-faux (7, 9) ; et
un moyen de réglage d'une position radiale des panneaux (13A, 13P) par rapport aux parties de support (7, 9),
dans laquelle les panneaux (13A, 13P) sont maintenus dans une position entre les parties de support en porte-à-faux (7, 9) par le moyen de réglage.

2. Emboîture selon la revendication 1, dans laquelle l'emboîture (1) est une emboîture de désarticulation du genou ou transfémorale pour un membre résiduel de désarticulation du genou ou transfémoral, respectivement.

3. Emboîture selon la revendication 1 ou 2, dans laquelle :
la première partie de support en porte-à-faux (7) s'étend autour d'une partie médiale de la circonférence de l'emboîture ;
le premier panneau (13A) s'étend le long d'une partie latérale antérieure de l'emboîture ;
le second panneau (13P) s'étend le long d'une partie latérale postérieure de l'emboîture ; et
la seconde partie de support en porte-à-faux (9) s'étend le long d'une partie latérale de l'emboîture.

4. Emboîture selon la revendication 1, 2 ou 3, dans laquelle les premier (13A) et second (13P) panneaux s'étendent de manière proximale au-delà de la seconde partie de support en porte-à-faux (9).

5. Emboîture selon l'une quelconque des revendications précédentes, dans laquelle les panneaux (13A, 13P) comprennent une enveloppe externe rigide ou semi-rigide (13AS, 13PS) et une partie interne compressible de manière élastique.

6. Emboîture selon la revendication 5, dans laquelle la partie interne comprend une bande d'éthylène-acétate de vinyle.

7. Emboîture selon l'une quelconque des revendications précédentes, dans laquelle en combinaison les parties de support en porte-à-faux (7, 9) et les panneaux (13A, 13P) s'étendent autour de la circonférence de l'emboîture.

8. Emboîture selon l'une quelconque des revendications précédentes, dans laquelle le moyen de réglage d'une position radiale des panneaux (13A, 13P) par rapport aux parties de support (7, 9) comprend un cordon ou des cordons (15D, 15P) traversant les parties de support et les panneaux.

9. Emboîture selon la revendication 8, dans laquelle le cordon est ou les cordons (15D, 15P) sont disposés sur une surface externe des parties de support en porte-à-faux (7, 9) ou intégrés dans une enveloppe externe des parties de support en porte-à-faux (7, 9) .

10. Emboîture selon la revendication 8 ou 9, dans laquelle les cordons (15D, 15P) comprennent un premier cordon (15D) et un second cordon (15P).

11. Emboîture selon la revendication 8, 9 ou 10, comprenant en outre un moyen pour tirer le cordon ou les cordons (15D, 15P) radialement vers l'intérieur.

12. Emboîture selon la revendication 11, dans laquelle le moyen pour tirer le cordon ou les cordons (15D, 15P) radialement vers l'intérieur comprend un mécanisme à rochet.

13. Emboîture selon la revendication 12 lorsqu'elle dépend de la revendication 10, dans laquelle le mécanisme à rochet comprend des première (17P) et seconde (17D) enrouleur à rochet, chaque enrouleur à rochet (17P, 17D) tirant l'un du premier et d'un second cordon (15D, 15P), les premier et second cordons (15D, 15P) étant espacés distalement et proximalement l'un de l'autre le long des parties de support en porte-à-faux (7, 9).

14. Emboîture selon la revendication 11, 12, 13, dans laquelle le moyen de étirage du cordon radialement vers l'intérieur comprend un moteur.

15. Emboîture (1) selon l'une quelconque des revendications précédentes et comprenant en outre :
une pluralité de capteurs (21) à l'intérieur de l'élément de support ; et un moyen d'adaptation d'un ou plusieurs de :
un volume intérieur de l'élément de support ;
une température à l'intérieur de l'élément de support ;
des niveaux d'oxygène à l'intérieur de l'élément de support ; et
une pression à l'intérieur de l'élément de support, et
des modules de communication pour la communication entre les capteurs (21) et le moyen d'adaptation.
